# EUROPEAN PATENT APPLICATION

(11) **EP 2 241 331 A2**
(43) Date of publication of application: **20.10.2010**
(21) Application number: 10007361.8
(22) Date of filing: 15.12.2004
(51) Int. Cl.: A61K 45/00, A61K 47/00, G01N 33/53, C07K 16/00, C12P 21/08

(54) **Novel anti-DC-SIGN antibodies**

(30) Priority: 15.12.2003 US 529517 P
(62) Divisional of application: 04814026.3
(71) Applicant: ALEXION PHARMACEUTICALS, INC., Cheshire, CT 06410 (US)
(72) Inventor: Bowdish, Katherine S., Del Mar, CA 92014 (US); Kretz-Rommel, Anke, San Diego, CA 92116 (US)
(74) Representative: Vossius & Partner

(57) **Abstract**

The present invention relates to an antibody for use in treating cancer, wherein the antibody binds to DC-SIGN on the surface of a cancer cell and wherein the binding of the antibody to a DC-SIGN-expressing cancer cell kills the cancer cell. Furthermore, the invention relates to an antibody for use in treating an inflammatory disease or an autoimmune disease, wherein the antibody binds to DC-SIGN on the surface of a dendritic cell and wherein the binding of the antibody to a DC-SIGN-expressing dendritic cell kills the dendritic cell.

SEQUENCE LISTING

<110>  Bowdish, Katherine S.
       Kretz-Rommel, Anke

<120>  NOVEL ANTI-DC-SIGN ANTIBODIES

<130>  M2151 EP/1

<150>  EP 04 81 4026.3
<151>  2004-12-15

<150>  US 60/529,517
<151>  2003-12-15

<160>  62

<170>  PatentIn version 3.2

<210>  1
<211>  25
<212>  DNA
<213>  artificial sequence

<220>
<223>  oligonucleotide

<400>  1
ctaactccat ggtgaccctg ggatg                                          25


<210>  2
<211>  24
<212>  DNA
<213>  artificial sequence

<220>
<223>  oligonucleotide

<400>  2
caactggctc ctcggtgact ctag                                           24


<210>  3
<211>  24
<212>  DNA
<213>  artificial sequence

<220>
<223>  oligonucleotide

<400>  3
cagtgagcag ttaacatctg gagg                                           24


<210>  4
<211>  62
<212>  DNA
<213>  artificial sequence

<220>
<223>  oligonucleotide

<220>
<221>  modified_base
<222>  (60)..(60)
<223>  um

<220>
<221>  misc_feature
<222>  (60)..(60)
<223>  3' - phosphorothioate

<220>
<221>  variation
<222>  60
<223>  /replace="c"
      /replace="g"
      /replace="t"

<220>
<221>  modified_base
<222>  (61)..(61)
<223>  cm

<220>
<221>  misc_feature
<222>  (61)..(61)
<223>  3' - phosphorothioate

<220>
<221>  modified_base
<222>  (62)..(62)
<223>  2' -o-methyladenosine

<220>
<221>  misc_feature
<222>  (62)..(62)
<223>  3'-phosphorothioate propyl-OH

<400>  4
gacgtggccg ttggaagagg agtgcctagg gttaccatgg agttagtttg ggcagcagaa     60

ca                                                                    62


<210>  5
<211>  62
<212>  DNA
<213>  artificial sequence

<220>
<223>  oligonucleotide

<220>
<221>  modified_base
<222>  (60)..(60)
<223>  cm

<220>
<221>  misc_feature
<222>  (60)..(60)
<223>  3' - phosphorothioate

<220>
<221>  modified_base
<222>  (61)..(61)
<223>  2' -O-methyladenosine

<220>
<221>  misc_feature
<222>  (61)..(61)
<223>  3' - phosphorothioate

<220>
<221>  modified_base

<222>  (62)..(62)
<223>  um

<220>
<221>  misc_feature
<222>  (62)..(62)
<223>  3' - phosphorothioate propyl-OH

<220>
<221>  variation
<222>  62
<223>  /replace="c"
       /replace="g"
       /replace="t"

<400>  5
gacgtggccg ttggaagagg agtgcctagg gtcatcgagg agccagttgt atctccacac      60

aa                                                                      62


<210>  6
<211>  65
<212>  DNA
<213>  artificial sequence

<220>
<223>  oligonucleotide

<220>
<221>  modified_base
<222>  (63)..(63)
<223>  2'-O-methyladenosine

<220>
<221>  misc_feature
<222>  (63)..(63)
<223>  3'-phosphorothioate

<220>
<221>  modified_base
<222>  (64)..(64)
<223>  um

<220>
<221>  misc_feature
<222>  (64)..(64)
<223>  3'-phosphorothioate

<220>
<221>  variation
<222>  64
<223>  /replace="c"
       /replace="g"
       /replace="t"

<220>
<221>  modified_base
<222>  (65)..(65)
<223>  um

<220>
<221>  misc_feature
<222>  (65)..(65)
<223>  3'-phosphorothioate propyl-OH

<220>
<221>  variation

<222>  65
<223>  /replace="c"
       /replace="g"
       /replace="t"

<400>  6
gacgaccggc taccaagagg agtgtccgga tgttaactgc tcactggatg gtgggaagat      60

ggaaa                                                                  65


<210>  7
<211>  24
<212>  DNA
<213>  artificial sequence

<220>
<223>  primer

<400>  7
gacgtggccg ttggaagagg agtg                                             24


<210>  8
<211>  24
<212>  DNA
<213>  artificial sequence

<220>
<223>  primer

<400>  8
gacgaccggc taccaagagg agtg                                             24


<210>  9
<211>  109
<212>  PRT
<213>  artificial sequence

<220>
<223>  majority of murine clones

<400>  9

Asp Ile Gln Met Thr Gln Thr Thr Ser Ser Leu Ser Ala Ser Leu Gly
1               5                   10                  15


Asp Arg Val Thr Ile Thr Cys Arg Ala Ser Gln Asp Ile Asn Asn Tyr
            20                  25                  30


Leu Asn Trp Tyr Gln Gln Lys Pro Asp Gly Thr Val Lys Leu Leu Ile
        35                  40                  45


Tyr Tyr Thr Ser Arg Leu His Ser Gly Val Pro Ser Arg Phe Ser Gly
    50                  55                  60


Ser Gly Ser Gly Thr Asp Tyr Ser Leu Thr Ile Ser Asn Leu Ala Gln
65                  70                  75                  80


Glu Asp Ile Ala Thr Tyr Phe Cys Gln Gln Gly Asp Thr Leu Pro Trp
                85                  90                  95

Thr Phe Gly Gly Gly Thr Lys Leu Glu Ile Lys Arg Ala
            100                 105

<210>  10
<211>  109
<212>  PRT
<213>  murine

<400>  10

Asp Ile Gln Met Thr Gln Ser Pro Ala Ser Leu Ser Ala Ser Val Gly
1               5                   10                  15

Glu Thr Ile Thr Ile Thr Cys Arg Ala Ser Glu Asn Ile His Asn Tyr
            20                  25                  30

Leu Ala Trp Tyr Gln Gln Asn Gln Gly Lys Ser Pro Gln Leu Leu Val
        35                  40                  45

Tyr Asn Ala Lys Thr Leu Ala Val Gly Val Pro Ser Arg Phe Ser Gly
    50                  55                  60

Ser Gly Ser Gly Thr Gln Phe Ser Leu Lys Ile Val Ser Leu Gln Pro
65                  70                  75                  80

Glu Asp Phe Gly Asn Tyr Tyr Cys Gln His Phe Trp Asn Thr Pro Trp
                85                  90                  95

Thr Phe Gly Arg Gly Thr Lys Leu Glu Ile Lys Arg Ala
            100                 105

<210>  11
<211>  109
<212>  PRT
<213>  murine

<400>  11

Asp Ile Gln Met Thr Gln Thr Thr Ser Ser Leu Ser Ala Ser Leu Gly
1               5                   10                  15

Asp Arg Val Thr Ile Thr Cys Arg Thr Ser Gln Asp Ile Asp Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Asn Asn Leu Ala Gln
65                  70                  75                  80

Glu Asp Ile Ala Thr Tyr Phe Cys Gln Gln Gly Asp Thr Leu Pro Phe

                85                  90                  95

Thr Phe Gly Ser Gly Thr Thr Leu Glu Ile Lys Arg Ala
            100                 105

<210> 12
<211> 109
<212> PRT
<213> murine

<400> 12

Asp Ile Pro Met Thr Gln Thr Thr Ser Ser Gln Ser Ala Ser Leu Gly
1               5                   10                  15

Asp Arg Val Thr Ile Thr Cys Arg Ala Ser Gln Asp Ile Asn Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Leu Val Thr Tyr Phe Cys Gln Gln Gly Lys Thr Leu Pro Trp
                85                  90                  95

Thr Phe Gly Gly Gly Thr Lys Leu Glu Ile Lys Arg Ala
            100                 105

<210> 13
<211> 109
<212> PRT
<213> murine

<400> 13

Asp Ile Gln Met Thr Gln Thr Thr Ser Ser Leu Ser Ala Ser Leu Gly
1               5                   10                  15

Asp Arg Val Thr Ile Ser Cys Arg Ala Ser His Asp Ile Asn Asp Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu Gln Ser Gly Val Pro Ser Arg Phe Arg Gly
    50                  55                  60

Tyr Gly Ser Gly Thr Asp Tyr Ser Leu Thr Ile Ser Asn Leu Ala Gln
65                  70                  75                  80

Glu Asp Phe Ala Thr Tyr Phe Cys Gln Gln Gly His Thr Leu Pro Tyr
                85                  90                  95

Thr Phe Gly Gly Gly Thr Lys Leu Glu Ile Lys Arg Ala
            100                 105

<210>  14
<211>  109
<212>  PRT
<213>  murine

<400>  14

Asp Ile Gln Met Thr Gln Thr Thr Ser Ser Leu Ser Ala Ser Leu Gly
1               5                   10                  15

Asp Arg Val Thr Ile Thr Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Ala Gln
65                  70                  75                  80

Glu Asp Ile Ala Thr Tyr Phe Cys Gln Gln Gly Asp Lys Leu Pro Phe
                85                  90                  95

Thr Phe Gly Ser Gly Thr Thr Leu Glu Ile Lys Arg Ala
            100                 105

<210>  15
<211>  109
<212>  PRT
<213>  murine

<400>  15

Asp Ile Gln Met Thr Gln Thr Thr Ser Ser Gln Ser Ala Ser Leu Gly
1               5                   10                  15

Asp Arg Val Thr Ile Thr Cys Arg Ala Ser Gln Asp Ile Asn Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln

65                  70                  75                  80

Glu Asp Leu Ala Thr Tyr Phe Cys Gln Gln Gly Lys Thr Leu Pro Trp
                85                  90                  95

Thr Phe Gly Gly Gly Thr Lys Leu Glu Ile Lys Arg Ala
            100                 105

<210>  16
<211>  109
<212>  PRT
<213>  murine

<400>  16

Asp Ile Gln Met Thr Gln Thr Thr Ser Ser Leu Phe Ala Ser Leu Gly
1               5                   10                  15

Asp Arg Val Thr Ile Ser Cys Arg Ala Ser Gln Asp Ile Arg Asn Asn
            20                  25                  30

Leu Asn Trp Tyr Gln Gln Lys Pro Asp Gly Thr Val Lys Leu Leu Ile
        35                  40                  45

Tyr Tyr Thr Ser Ser Leu Pro Ser Gly Val Pro Ser Arg Phe Ser Gly
    50                  55                  60

Ser Arg Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asp Thr Leu Pro Pro
                85                  90                  95

Thr Phe Gly Gly Gly Thr Lys Leu Glu Ile Lys Arg Ala
            100                 105

<210>  17
<211>  109
<212>  PRT
<213>  murine

<400>  17

Asp Ile Gln Met Thr Gln Thr Thr Ser Ser Leu Ser Ala Ser Leu Gly
1               5                   10                  15

Asp Arg Val Thr Ile Ser Cys Arg Ala Ser His Asp Ile Asn Asp Tyr
            20                  25                  30

Leu Asn Trp Tyr Gln Gln Lys Pro Asp Gly Thr Val Lys Leu Leu Ile
        35                  40                  45

Tyr Tyr Thr Ser Ser Leu Gln Ser Gly Val Pro Ser Arg Phe Arg Gly
    50                  55                  60

Tyr Gly Ser Gly Thr Asp Tyr Ser Leu Thr Ile Ser Asn Leu Ala Gln
65                  70                  75                  80

Glu Asp Phe Ala Thr Tyr Phe Cys Gln Gln Gly His Thr Leu Pro Tyr
                85                  90                  95

Thr Phe Gly Gly Gly Thr Lys Leu Glu Ile Lys Arg Ala
            100                 105

<210>  18
<211>  117
<212>  PRT
<213>  murine

<400>  18

Glu Val Gln Leu Gln Gln Ser Gly Pro Gly Leu Val Lys Pro Ser Gln
1               5                   10                  15

Ser Leu Ser Leu Ala Cys Ser Val Thr Gly Tyr Ser Ile Thr Ser Gly
            20                  25                  30

Tyr Tyr Trp Asn Trp Ile Arg Gln Ser Pro Gly Asn Lys Leu Glu Trp
        35                  40                  45

Met Gly Tyr Ile Ser Tyr Asp Gly Asn Ser Asp Tyr Asn Pro Ser Phe
    50                  55                  60

Lys Asn Arg Ile Ser Ile Thr Arg Asp Thr Ser Lys Asn Gln Phe Phe
65                  70                  75                  80

Leu Arg Leu Asn Ser Leu Thr Thr Glu Asp Thr Ala Thr Tyr Tyr Cys
                85                  90                  95

Val Arg Asp Asp Ser Gly Arg Phe Pro Gln Trp Gly Gln Gly Thr Leu
            100                 105                 110

Val Thr Val Ser Ala
        115

<210>  19
<211>  121
<212>  PRT
<213>  artificial sequence

<220>
<223>  majority of murine clones

<400>  19

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Tyr Met His Trp Val Lys Gln Ser His Val Lys Ser Leu Glu Trp Ile
        35                  40                  45

Gly Arg Ile Asn Pro Tyr Asn Gly Ala Thr Ser Tyr Asn Gln Asn Phe
    50                  55                  60

Lys Asp Lys Ala Ser Leu Thr Val Asp Lys Ser Ser Thr Thr Val Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Thr Ser Val Thr Val Ser Ser
        115                 120

<210>  20
<211>  121
<212>  PRT
<213>  murine

<400>  20

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Tyr Met His Trp Val Lys Gln Ser His Val Lys Ser Leu Glu Trp Ile
        35                  40                  45

Gly Arg Ile Asn Pro Tyr Asn Gly Ala Thr Ser Tyr Asn Gln Asn Phe
    50                  55                  60

Lys Asp Lys Ala Ser Leu Thr Val Asp Lys Ser Ser Thr Thr Val Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Ser Ser Val Ala Val Ser Ser
        115                 120

<210>  21
<211>  121
<212>  PRT

<213>  murine

<400>  21

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Tyr Met His Trp Val Lys Gln Ser His Val Lys Ser Leu Glu Trp Ile
        35                  40                  45

Gly Arg Ile Asn Pro Tyr Asn Gly Ala Thr Tyr Tyr Asn His Asn Phe
    50                  55                  60

Lys Asp Lys Ala Thr Leu Thr Val His Lys Ser Ser Thr Thr Val Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Thr Ser Val Thr Val Ser Ser
        115                 120

<210>  22
<211>  121
<212>  PRT
<213>  murine

<400>  22

Glu Val Gln Leu Gln Gln Ser Gly Pro Glu Leu Val Lys Pro Gly Thr
1               5                   10                  15

Ser Val Lys Ile Ser Cys Lys Ala Ser Gly Tyr Ser Phe Thr Gly Tyr
            20                  25                  30

Tyr Ile His Trp Val Arg Gln Arg His Val Lys Ser Leu Glu Trp Ile
        35                  40                  45

Gly Arg Ile Asn Pro Tyr Ser Gly Ala Thr Ser Tyr Asn Gln Ser Phe
    50                  55                  60

Lys Asp Lys Ala Ser Leu Thr Val Asp Lys Ser Ser Thr Thr Ala Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly

            100                 105                 110

Gln Gly Thr Ser Val Thr Val Ser Ser
        115                 120

<210> 23
<211> 121
<212> PRT
<213> murine

<400> 23

Glu Val Gln Leu Gln Gln Ser Gly Pro Glu Leu Val Lys Pro Gly Thr
1               5                   10                  15

Ser Val Lys Ile Ser Cys Lys Ala Ser Gly Tyr Ser Phe Thr Gly Tyr
            20                  25                  30

Tyr Ile His Trp Val Arg Gln Arg His Val Lys Ser Leu Glu Trp Ile
        35                  40                  45

Gly Arg Ile Asn Pro Tyr Ser Gly Ala Thr Ser Tyr Asn Gln Ser Phe
    50                  55                  60

Lys Asp Lys Ala Ser Leu Thr Val Asp Lys Ser Ser Thr Thr Ala Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Thr Ser Val Thr Val Ser Ser
        115                 120

<210> 24
<211> 121
<212> PRT
<213> murine

<400> 24

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Tyr Met His Trp Val Lys Gln Ser His Val Lys Ser Leu Glu Trp Ile
        35                  40                  45

Gly Arg Ile Asn Pro Tyr Asn Gly Ala Thr Ser Tyr Asn Gln Asn Phe
    50                  55                  60

Lys Asp Lys Ala Ser Leu Thr Val Asp Lys Ser Ser Thr Thr Val Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Thr Ser Val Thr Val Ser Ser
        115                 120

<210>  25
<211>  121
<212>  PRT
<213>  murine

<400>  25

Glu Val Gln Leu Gln Gln Ser Gly Pro Glu Leu Val Lys Pro Gly Ala
1               5                   10                  15

Ser Val Lys Ile Ser Cys Lys Ala Ser Gly Tyr Ser Phe Thr Gly Tyr
            20                  25                  30

Tyr Met His Trp Val Lys Gln Ser His Val Lys Ser Leu Glu Trp Ile
        35                  40                  45

Gly Arg Ile Asn Pro Tyr Asn Gly Ala Pro Ser Tyr Asn Gln Asn Phe
    50                  55                  60

Lys Asp Lys Ala Ser Leu Thr Val Asp Glu Ser Ser Thr Thr Val Tyr
65                  70                  75                  80

Met Glu Val His Ser Leu Thr Ser Glu Asp Ser Ala Val Tyr Tyr Cys
                85                  90                  95

Val Arg Ser Asn Asp Gly Tyr Tyr Ser Tyr Pro Met Asp Tyr Trp Gly
            100                 105                 110

Gln Gly Thr Ser Val Thr Val Ser Ser
        115                 120

<210>  26
<211>  116
<212>  PRT
<213>  murine

<400>  26

Glu Val Gln Leu Gln Gln Ser Gly Ala Glu Leu Val Arg Pro Gly Ala
1               5                   10                  15

Leu Val Lys Leu Ser Cys Lys Ala Ser Gly Phe Asn Ile Lys Asp Tyr

20                  25                  30

Tyr Ile His Trp Val Lys Gln Arg Pro Glu Gln Gly Leu Glu Trp Ile
        35                  40                  45

Gly Trp Ile Asp Pro Glu Asn Gly Asn Thr Ile Tyr Asp Pro Lys Phe
    50                  55                  60

Gln Gly Lys Ala Ser Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Arg Tyr Tyr Leu Gly Val Asp Tyr Trp Gly Gln Gly Thr Ser Val
            100                 105                 110

Thr Val Ser Ser
        115

<210>  27
<211>  121
<212>  PRT
<213>  murine

<400>  27

Glu Val Gln Leu Gln Gln Ser Gly Pro Asp Leu Val Lys Pro Gly Ala
1               5                   10                  15

Ser Val Arg Ile Ser Cys Lys Thr Ser Gly Phe Thr Phe Ala Asn Tyr
            20                  25                  30

Tyr Ile His Trp Val Lys Gln Arg Pro Gly Gln Gly Leu Glu Trp Ile
        35                  40                  45

Gly Trp Ile Phe Pro Gly Asn Phe Lys Thr Glu Tyr Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Gly Tyr Gly Tyr Ala Val Asp Tyr Trp Gly Gln Gly Thr Ser
            100                 105                 110

Val Thr Val Ser Ser Ala Lys Thr Thr
        115                 120

<210>  28
<211>  120

<212>  PRT
<213>  murine

<400>  28

Glu Val Gln Leu Gln Gln Ser Gly Ala Glu Leu Val Lys Pro Gly Ala
1               5                   10                  15

Ser Val Lys Leu Ser Cys Thr Ala Ser Gly Phe Asn Ile Lys Asp Thr
            20                  25                  30

Tyr Met His Trp Val Lys Gln Arg Pro Glu Gln Gly Leu Glu Trp Ile
        35                  40                  45

Gly Arg Ile Asp Pro Ala Asn Gly Asn Thr Lys Tyr Asp Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Ala Arg Asp Tyr Tyr Gly Ile Tyr Val Asp Tyr Trp Gly Gln Gly Thr
            100                 105                 110

Thr Leu Thr Val Ser Ser Ala Lys
        115                 120

<210>  29
<211>  122
<212>  PRT
<213>  murine

<400>  29

Glu Val Gln Leu Gln Gln Ser Gly Ala Glu Leu Ala Arg Pro Gly Ala
1               5                   10                  15

Ser Val Lys Leu Ser Cys Lys Ala Ser Gly Tyr Thr Phe Arg Ser Tyr
            20                  25                  30

Trp Met Gln Trp Val Lys Gln Arg Pro Gly Gln Gly Leu Glu Trp Ile
        35                  40                  45

Gly Ala Ile Tyr Pro Gly Asp Gly Asp Thr Arg Tyr Thr Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Ala Ser Glu Asp Ser Ala Val Tyr Phe Cys
                85                  90                  95

Ala Arg Gly Gly Leu Lys Ser Phe Tyr Ala Met Asp His Trp Gly Gln
            100                 105                 110

Gly Thr Ser Val Thr Val Ser Ser Ala Lys
        115                 120

<210>  30
<211>  120
<212>  PRT
<213>  murine

<400>  30

Glu Val Gln Leu Gln Gln Ser Gly Pro Asp Leu Val Lys Pro Gly Ala
1               5                   10                  15

Ser Val Arg Ile Ser Cys Lys Thr Ser Gly Phe Thr Phe Ala Ser Tyr
            20                  25                  30

Tyr Ile His Trp Val Lys Gln Arg Pro Gly Gln Gly Leu Glu Trp Ile
        35                  40                  45

Gly Trp Ile Phe Pro Gly Asn Phe Lys Thr Glu Tyr Asn Glu Lys Phe
    50                  55                  60

Lys Gly Lys Ala Thr Leu Thr Ala Asp Arg Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Gly Tyr Gly Tyr Ala Val Asp Tyr Trp Gly Gln Gly Thr Ser
            100                 105                 110

Val Thr Val Ser Ser Ala Lys Thr
        115                 120

<210>  31
<211>  116
<212>  PRT
<213>  murine

<400>  31

Glu Val Gln Leu Gln Gln Ser Gly Pro Asp Leu Val Lys Pro Gly Ala
1               5                   10                  15

Ser Val Arg Ile Ser Cys Lys Ala Ser Gly Phe Thr Phe Ala Ser Tyr
            20                  25                  30

Tyr Ile His Trp Val Lys Gln Arg Pro Gly Gln Gly Leu Glu Trp Ile
        35                  40                  45

Gly Trp Ile Phe Pro Gly Asn Asn Asn Thr Lys Ser Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Gly Tyr Gly Tyr Ala Val Asp Tyr Trp Gly Gln Gly Thr Ser
            100                 105                 110

Val Thr Val Ser
        115

<210>  32
<211>  120
<212>  PRT
<213>  murine

<400>  32

Glu Val Gln Leu Gln Gln Ser Gly Pro Asp Leu Val Lys Pro Gly Ala
1               5                   10                  15

Ser Val Arg Ile Ser Cys Lys Ala Ser Gly Phe Thr Phe Ala Ser Tyr
            20                  25                  30

Tyr Ile His Trp Val Lys Gln Arg Pro Gly Gln Gly Leu Glu Trp Ile
        35                  40                  45

Gly Trp Ile Phe Pro Gly Asn Asn Asn Thr Lys Ser Asn Glu Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Gly Tyr Gly Tyr Ala Val Asp Tyr Trp Gly Gln Gly Thr Ser
            100                 105                 110

Val Thr Val Ser Ser Ala Lys Thr
        115                 120

<210>  33
<211>  118
<212>  PRT
<213>  murine

<400>  33

Glu Val Gln Leu Gln Gln Ser Gly Ala Glu Leu Val Lys Pro Gly Ala
1               5                   10                  15

Ser Val Lys Leu Ser Cys Thr Ala Ser Asp Phe Asn Ile Lys Asp Thr
            20                  25                  30

Tyr Ile Gln Trp Val Lys Gln Arg Pro Glu Gln Gly Leu Glu Trp Val
        35                  40                  45

Gly Arg Ile Asp Pro Ala Asn Gly Glu Ile Lys Tyr Asp Pro Lys Phe
    50                  55                  60

Gln Gly Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Tyr Cys
                85                  90                  95

Thr Ala Tyr Phe Leu Val Tyr Trp Gly Gln Gly Thr Leu Val Thr Val
            100                 105                 110

Ser Ala Ala Lys Thr Thr
        115

<210> 34
<211> 120
<212> PRT
<213> murine

<400> 34

Glu Val Gln Leu Lys Glu Ser Gly Pro Gly Leu Val Ala Pro Ser Gln
1               5                   10                  15

Ser Leu Ser Ile Thr Cys Thr Val Ser Gly Phe Ser Leu Ser Arg Tyr
            20                  25                  30

Thr Val His Trp Ile Arg Gln Pro Pro Gly Lys Gly Leu Glu Trp Leu
        35                  40                  45

Gly Met Ile Trp Gly Gly Gly Thr Thr Asp Tyr Asn Ser Ala Leu Lys
    50                  55                  60

Ser Arg Leu Ser Ile Ser Lys Asp Asn Ser Glu Ser Gln Val Phe Leu
65                  70                  75                  80

Lys Met Asp Ser Leu Gln Thr Asp Asp Thr Ala Lys Tyr Tyr Cys Ala
                85                  90                  95

Arg Ile Asn Phe Gly Ile Leu Gly Tyr Trp Gly Gln Gly Thr Leu Val
            100                 105                 110

Thr Val Ser Ala Ala Lys Thr Thr
        115                 120

<210> 35

<211>  121
<212>  PRT
<213>  murine

<400>  35

Glu Val Gln Leu Gln Gln Ser Gly Thr Glu Leu Val Arg Pro Gly Ala
1               5                   10                  15

Leu Val Lys Leu Ser Cys Lys Ala Ser Gly Phe Asn Ile Lys Asp Tyr
            20                  25                  30

Phe Met His Trp Val Lys Gln Arg Pro Glu Gln Gly Leu Glu Trp Ile
        35                  40                  45

Gly Trp Ile Asp Pro Lys Asn Gly Asn Thr Ile Tyr Asp Pro Lys Phe
    50                  55                  60

Gln Val Lys Ala Ser Ile Thr Ala Asp Thr Ser Ser Asn Thr Ala Tyr
65                  70                  75                  80

Leu Gln Leu Thr Ser Leu Thr Ser Glu Asp Thr Ala Val Tyr Tyr Cys
                85                  90                  95

Thr Thr Thr Tyr Pro Asn Ala Leu Asp Tyr Trp Gly Gln Gly Thr Ser
            100                 105                 110

Val Thr Val Ser Ser Ala Lys Thr Thr
        115                 120

<210>  36
<211>  108
<212>  PRT
<213>  murine

<400>  36

Asp Ile Ala Met Thr Gln Ser His Lys Phe Met Ser Thr Pro Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Ser Ala Ser Tyr Arg Tyr Thr Gly Val Pro Asp Arg Phe Thr Gly
    50                  55                  60

Ser Gly Ser Gly Thr Asp Phe Thr Phe Thr Ile Ser Ser Val Gln Ala
65                  70                  75                  80

Glu Asp Leu Ala Val Tyr Tyr Cys Gln Gln His Tyr Ile Thr Pro Leu
                85                  90                  95

Thr Phe Gly Ala Gly Thr Lys Leu Glu Leu Lys Arg
            100                 105

<210>  37
<211>  113
<212>  PRT
<213>  murine

<400>  37

Asp Val Val Met Thr Gln Thr Pro Leu Thr Leu Ser Val Thr Ile Gly
1               5                   10                  15

Gln Pro Ala Ser Ile Ser Cys Lys Ser Ser Gln Ser Leu Leu Asp Ser
            20                  25                  30

Asp Gly Lys Thr Tyr Leu Asn Trp Leu Leu Trp Arg Pro Gly Gln Ser
        35                  40                  45

Pro Lys Arg Leu Ile Tyr Leu Val Ser Lys Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Phe Cys Trp Gln Asp
                85                  90                  95

Thr His Phe Pro His Val His Val Arg Cys Trp Asp Gln Ala Gly Thr
            100                 105                 110

Thr

<210>  38
<211>  108
<212>  PRT
<213>  murine

<400>  38

Ser Ile Val Leu Thr Gln Ser Pro Lys Ser Met Ser Met Ser Val Gly
1               5                   10                  15

Glu Arg Val Thr Leu Ser Cys Lys Ala Ser Glu Asn Val Gly Thr Tyr
            20                  25                  30

Val Ser Trp Tyr Gln Gln Lys Pro Asp Gln Ser Pro Lys Leu Leu Ile
        35                  40                  45

Tyr Gly Ser Ser Asn Arg Ser Thr Gly Val Pro Asp Arg Phe Thr Gly
    50                  55                  60

Ser Gly Ser Ala Thr Asp Phe Thr Leu Thr Ile Ser Ser Val Gln Ala

65                  70                  75                  80

Glu Asp Leu Ala Asp Tyr His Cys Gly Gln Ser Tyr Asn Tyr Pro Pro
                85                  90                  95

Thr Phe Gly Gly Gly Thr Lys Leu Glu Ile Lys Arg
            100                 105

<210>  39
<211>  108
<212>  PRT
<213>  murine

<400>  39

Asp Ile Val Leu Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Lys Pro Gly Gln Ser Pro Lys Leu Leu Ile
        35                  40                  45

Tyr Trp Ala Ser Thr Arg His Thr Gly Val Pro Asp Arg Phe Thr Gly
    50                  55                  60

Ser Gly Ser Gly Thr Asp Tyr Thr Leu Thr Ile Ser Ser Val Gln Ala
65                  70                  75                  80

Glu Asp Leu Ala Leu Tyr Tyr Cys Gln Gln Tyr Tyr Ser Thr Pro Arg
                85                  90                  95

Thr Phe Gly Gly Gly Thr Lys Leu Glu Ile Lys Arg
            100                 105

<210>  40
<211>  108
<212>  PRT
<213>  murine

<400>  40

Arg Tyr Gln Met Thr Gln Thr Thr Ser Ser Leu Ser Ala Ser Leu Gly
1               5                   10                  15

Asp Arg Val Thr Ile Ser Cys Ser Ala Ser Gln Asp Ile Thr Asn Tyr
            20                  25                  30

Leu Asn Trp Tyr Gln Gln Lys Pro Asp Gly Thr Val Lys Leu Leu Leu
        35                  40                  45

Tyr Tyr Thr Ser Thr Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Val Ala Thr Tyr Tyr Cys Gln Gln Tyr Gly Asn Leu Pro Tyr
                85                  90                  95

Thr Phe Gly Gly Gly Thr Lys Leu Glu Ile Lys Arg
            100                 105

<210>  41
<211>  108
<212>  PRT
<213>  murine

<400>  41

Asp Ile Gln Cys Thr Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Ala Asp Ile Ala Thr Tyr Phe Cys Gln Gln Gly Lys Thr Leu Pro Trp
                85                  90                  95

Thr Phe Gly Gly Gly Thr Lys Leu Glu Ile Lys Arg
            100                 105

<210>  42
<211>  108
<212>  PRT
<213>  murine

<400>  42

Arg His Gln Met Thr Gln Ser His Lys Phe Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Gln Asn Pro Gly Gln Ser Leu Lys Leu Leu Ile
        35                  40                  45

Tyr Trp Ala Ser Thr Arg His Thr Gly Val Pro Asp Arg Phe Thr Gly

50                  55                  60

Ser Gly Ser Gly Thr Asp Tyr Thr Leu Thr Ile Asn Ser Val Gln Ala
65                  70                  75                  80

Glu Asp Leu Thr Leu Tyr Tyr Cys Gln Gln His Tyr Ile Thr Pro Leu
                85                  90                  95

Thr Phe Gly Ala Gly Thr Lys Leu Glu Leu Lys Arg
            100                 105

<210>  43
<211>  108
<212>  PRT
<213>  murine

<400>  43

Arg Tyr Pro Asp Ala Gln Thr Thr Ser Ser Leu Ser Ala Ser Leu Gly
1               5                   10                  15

Asp Arg Val Thr Ile Ser Cys Arg Ala Ser Gln Asp Ile Ser Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Arg Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Pro
                85                  90                  95

Thr Phe Gly Gly Gly Thr Lys Leu Glu Ile Lys Arg
            100                 105

<210>  44
<211>  112
<212>  PRT
<213>  murine

<400>  44

Asp Ile Val Gln Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asn Leu His Ser Gly Val Pro Ser
    50                  55                  60

Arg Phe Ser Gly Ser Gly Ser Gly Thr Asp Tyr Ser Leu Thr Ile Ser
65                  70                  75                  80

Asn Leu Glu Gln Ala Asp Ile Ala Thr Tyr Phe Cys Gln Gln Gly Lys
                85                  90                  95

Thr Leu Pro Trp Thr Phe Gly Gly Gly Thr Lys Leu Glu Ile Lys Arg
            100                 105                 110

<210>  45
<211>  9
<212>  PRT
<213>  murine

<400>  45

Gln His Phe Trp Asn Thr Pro Trp Thr
1               5

<210>  46
<211>  9
<212>  PRT
<213>  murine

<400>  46

Gln Gln Gly His Thr Leu Pro Tyr Thr
1               5

<210>  47
<211>  7
<212>  PRT
<213>  murine

<400>  47

Ser Asn Asp Gly Tyr Tyr Ser
1               5

<210>  48
<211>  7
<212>  PRT
<213>  murine

<400>  48

Arg Tyr Tyr Leu Gly Val Asp
1               5

<210>  49
<211>  7
<212>  PRT
<213>  murine

<400>  49

Asp Asp Ser Gly Arg Phe Pro
1               5


<210>  50
<211>  7
<212>  PRT
<213>  murine

<400>  50

Tyr Gly Tyr Ala Val Asp Tyr
1               5


<210>  51
<211>  8
<212>  PRT
<213>  murine

<400>  51

Tyr Tyr Gly Ile Tyr Val Asp Tyr
1               5


<210>  52
<211>  4
<212>  PRT
<213>  murine

<400>  52

Phe Leu Val Tyr
1


<210>  53
<211>  7
<212>  PRT
<213>  murine

<400>  53

Asn Phe Gly Ile Leu Gly Tyr
1               5


<210>  54
<211>  7
<212>  PRT
<213>  murine

<400>  54

Tyr Pro Asn Ala Leu Asp Tyr
1               5


<210>  55
<211>  10
<212>  PRT
<213>  murine

<400>  55

Gly Leu Lys Ser Phe Tyr Ala Met Asp His
1               5                   10

<210>  56
<211>  9
<212>  PRT
<213>  murine

<400>  56

Gln Gln Gly Lys Thr Leu Pro Trp Thr
1               5


<210>  57
<211>  9
<212>  PRT
<213>  murine

<400>  57

Gln Gln Gly Asn Thr Leu Pro Pro Thr
1               5


<210>  58
<211>  9
<212>  PRT
<213>  murine

<400>  58

Gln Gln His Tyr Ile Thr Pro Leu Thr
1               5


<210>  59
<211>  9
<212>  PRT
<213>  murine

<400>  59

Gln Gln Tyr Gly Asn Leu Pro Tyr Thr
1               5


<210>  60
<211>  9
<212>  PRT
<213>  murine

<400>  60

Gln Gln Tyr Tyr Ser Thr Pro Arg Thr
1               5


<210>  61
<211>  9
<212>  PRT
<213>  murine

<400>  61

Gly Gln Ser Tyr Asn Tyr Pro Pro Thr
1               5

<210>  62
<211>  9
<212>  PRT
<213>  murine

<400>  62

Trp Gln Asp Thr His Phe Pro His Val
1               5

## Description

### RELATED APPLICATIONS

This application claims priority to U.S. Provisional Application Serial No. 60/529,517 filed December 15, 2003, the disclosure of which is incorporated herein in its entirety.

### TECHNICAL FIELD

The present disclosure relates to compositions useful in modulating, e.g., increasing or reducing, the immune response in an animal.

### BACKGROUND

Dendritic cells (DC) are professional antigen-presenting cells that capture antigens in the peripheral tissues and migrate via lymph or blood to the T cell area of the draining lymph nodes and spleen. They present processed antigens to naïve T cells, initiating antigen-specific primary T cell responses. DC are unique in their ability to interact with and activate resting T cells.

Naive T cells are characterized by a high expression of ICAM-3 which is a member of the IgG supergene family and is rapidly downregulated after activation (Vazeux et al., "Cloning and characterization of a new intercellular adhesion molecule ICAM-R", Nature, 360, pp 485-488 (1992)).

C-type lectins are calcium-dependent carbohydrate binding proteins with a wide range of biological functions, many of which are related to immunity. Recently, a novel ICAM-3 binding C-type lectin, known as DC-Specific ICAM-3 grabbing non-integrin, or DC-SIGN, was found. DC-SIGN is expressed on DCs and appears to mediate adhesion between dendritic cells and ICAM-3 on naive T cells and to be essential for DC-induced T cell proliferation (Geijtenbeek et al., "Identification of DC-SIGN, a novel dendritic cell-specific ICAM-3 receptor that supports primary immune responses", Cell, vol. 100, no. 5, pp. 575-585 (2000); Steinman, "DC-SIGN: A guide to some mysteries of dendritic cells", Cell, vol. 100, no. 5, pp. 491-494 (2000)). Binding of antibodies to DC-SIGN can result in internalization (Engering, et al., "The dendritic cell-specific adhesion receptor DC-SIGN internalizes antigen for presentation to T cells," J Immunol 168:2118 (2002)).

WO 00/63251, the contents of which are incorporated by reference herein, discloses that immune responses can be inhibited or prevented by preventing the interaction of DC-SIGN on dendritic cells with receptors on T cells, e.g., by using antibodies specific for DC-SIGN. Alternatively, an immune response to an antigen can be potentiated by binding an antigen to DC-SIGN on dendritic cells such that the antigen plus DC-SIGN is taken up by dendritic cells and processed and presented to T cells.

Besides its prominent role in DC-T cell clustering and initiation of T cell responses, DC-SIGN is a major receptor involved in infection of DC and subsequent transmission to T cells of viruses such as HIV-1, HIV-2, SIV-1, hepatitis C virus (HCV), Ebola virus, SARS, cytomegalovirus (CMV), Sindbis, and Dengue virus; bacteria such as Helicobacter pylori, Klebsiella pneumonae, and bacteria of the Mycobacterium genus, including M. tuberculosis and M. bovis; yeast such as Candida albicans; and parasites such as Leishmania pifanoi and Schistosoma mansoni.

Due to their position in the body surface as immunosurveillance cells, it is likely that DC are the first cells infected with these viruses after mucosal exposure and therefore play an important role in the immunopathogenesis of diseases caused by these viruses. It is now generally believed that these viruses, such as HIV, convert the normal trafficking process of DC to gain entry into lymph nodes and access to CD4⁺ T cells. For example, productive infection of DC with HIV-1 has been reported by several investigators (Granelli-Piperno et al., J Virol 72(4), 2733-7 (1998); Blauvelt et al., Nat Med 3(12), 1369-75 (1997)), and substantial evidence indicates that DC pulsed with HIV-1 in vitro can induce a vigorous infection when co-cultured with T cells (Cameron et al., Science 257(5068), 383-7 (1992)). Although it is still unclear whether a similar scenario occurs in HIV infected individuals, HIV-1 transmission from DC to T cells could contribute to the CD4⁺ T cell depletion observed in AIDS. Thus these viruses, such as HIV-1, and resting T cells exploit a similar highly expressed receptor to interact with DC.

Specific compositions useful in increasing or reducing the immune response in an animal remain desirable.

### SUMMARY

The present disclosure is directed to novel anti-DC-SIGN antibodies which can be useful in modulating the immune response of an animal.

In one embodiment, the anti-DC-SIGN antibodies interfere with the interaction of DC-SIGN expressing cells and ICAM-expressing cells. More specifically, in this embodiment the anti-DC-SIGN antibodies reduce the adhesion of C-type lectin receptors on the surface of dendritic cells to ICAM receptors on the surface of T cells. By modulating this adhesion, dendritic cell-T cell interactions can be influenced. Such interactions include cluster formation and antigen presentation, as well as primary T cell responses dependant thereon, resulting in a modulation of the immune response.

In another embodiment, the anti-DC-SIGN antibodies influence the migration of DC-SIGN expressing cells.

In another embodiment, the anti-DC-SIGN antibodies of the present disclosure can act as an agonist thereby enhancing T-cell response in an animal.

In another embodiment, the anti-DC-SIGN antibodies of the present disclosure may also be used to enhance the immune response to specific peptides, especially antigens. In such a case the anti-DC-SIGN antibodies are attached to a peptide and the combination of the two are administered to an animal. The antibodies direct the peptide to dendritic cells, which internalize the peptide and then present it on the dendritic cell surface to T cells, thereby generating an immune response to the peptide. In this case the antibodies can be useful as vaccines, including cancer vaccines.

In yet another embodiment, the anti-DC-SIGN antibodies of the present disclosure can be labeled with a toxin to DC-SIGN expressing cells. Administration of the anti-DC-SIGN antibodies labeled with toxin can then be utilized to reduce the levels of DC-SIGN expressing cells which, in some instances, can be beneficial, such as in the treatment of autoimmune disease.

In another embodiment, the anti-DC-SIGN antibodies of the present disclosure inhibit infection of dendritic cells by viruses such as HIV-1, HIV-2, SIV-1, hepatitis C virus (HCV), Ebola, SARS, cytomegalovirus (CMV), Sindbis, and Dengue; bacteria such as Helicobacter pylori, Klebsiella pneumonae, and bacteria of the genus Mycobacterium, including M. tuberculosis and M. bovis; yeast such as Candida albicans; and parasites such as Leishmania pifanoi and Schistosoma mansoni.

In some embodiments the anti-DC-SIGN antibodies of the present disclosure can be utilized as vaccines to diseases caused by the above-referenced viruses. In a further embodiment, the anti-DC-SIGIvT antibodies of the present disclosure can be used in the treatment of HIV-infections and similar disorders of the immune system, as well as to modulate the immune response to grafts or after transplant.

The anti-DC-SIGN antibodies of the present disclosure may also be utilized as routine diagnostics for tumor types associated with DC-SIGN expression and, in some embodiments, may be provided as part of diagnostic kits.

The anti-DC-SIGN antibodies of the present disclosure may also be utilized as therapeutics for the treatment of cancer and tumor types associated with DC-SIGN expression.

In some embodiments the anti-DC-SIGN antibodies of the present disclosure can be a humanized antibody. In other embodiments, the anti-DC-SIGN antibodies of the present disclosure can be an scFv.

In yet other embodiments, the anti-DC-SIGN antibodies of the present disclosure may also bind to L-SIGN.

Further embodiments of the present disclosure relate to prophylactic techniques as well as diagnostic techniques using the compositions and/or embodying the methods as described above. Compositions comprising the anti-DC-SIGN antibodies of the present disclosure in a pharmaceutically acceptable carrier are also provided.

### BRIEF DESCRIPTION OF THE FIGURES

FIGs. 1A and 1B are graphical depictions of the results of in vitro experiments in accordance with the present disclosure showing the reactivity of IgG1 clones and IgG2a clones, respectively, with human DC-SIGN.
FIG. 2 is a graphical depiction of the results of in vitro experiments in accordance with the present disclosure showing the reactivity of IgG1 clones and IgG2a clones with human L-SIGN and DC-SIGN.
FIG. 3 is a graphical depiction of the results of FACS analysis of 3 clones obtained in experiments in accordance with the present disclosure showing the reactivity of the clones with DC-SIGN on the surface of dendritic cells.
FIGs. 4a-4c provide the amino acid sequences of heavy chain clones and light chain clones obtained in experiments in accordance with the present disclosure that are reactive with DC-SIGN.
FIGs. 5A-5B are graphical depictions of the results of in vitro experiments in accordance with one embodiment of the present disclosure showing the reactivity of IgG1 clones and IgG2a clones, respectively, competing with AZN-D1 for binding to DC-SIGN.
FIG. 6 is a graphical depiction of the results of in vitro experiments in accordance with one embodiment of the present disclosure showing strong inhibition of ICAM binding to DC-SIGN by certain IgG1 clones.

### DETAILED DESCRIPTION OF THE PREFERRED EMBODIMENT

The present disclosure is based on the finding that antibodies directed against DC-SIGN can modulate the interaction of T cells with dendritic cells. In general, the anti-DC-SIGN antibodies of the present disclosure can bind, adhere to (preferably in a reversible manner), or serve as a ligand for DC-SIGN or natural variants or equivalents thereof. In some embodiments, the anti-DC-SIGN antibodies of the present disclosure may also bind L-SIGN.

The amino acid sequence of DC-SIGN is known and reported, for example, as shown in SEQ ID No. 1 and Figure 9 of WO OO/63251.

According to the present disclosure, the anti-DC-SIGN antibodies preferably include an antibody directed against DC-SIGN, or a part, fragment or epitope of DC-SIGN. As used herein, the term antibodies includes polyclonal, monoclonal, chimeric and single chain antibodies, as well as fragments (Fab, Fv, scFv, Fc) and Fab expression libraries. Such antibodies against DC-SIGN can be obtained as described hereinbelow or in any other manner known per se, such as those described in WO 95/32734, WO 96/23882, WO 98/02456, WO 98/41633 and/or WO 98/49306.

For instance, polyclonal antibodies can be obtained by immunizing a suitable host such as a goat, rabbit, sheep, rat, pig or mouse with DC-SIGN or an immunogenic portion, fragment or fusion thereof, optionally with the use of an immunogenic carrier (such as bovine serum albumin) and/or an adjuvant such as Freund's, saponin, ISCOM's, aluminum hydroxide or a similar mineral gel, or keyhole limpet hemocyanin or a similar surface active substance. After an immunoresponse against DC-SIGN has been raised (usually within 1-7 days), the antibodies can be isolated from blood or serum taken from the immunized animal in a manner known per se, which optionally may involve a step of screening for an antibody with desired properties (i.e. specificity) using known immunoassay techniques, for which reference is again made to WO 96/23882.

Monoclonal antibodies may be produced using continuous cell lines in culture, including hybridoma and similar techniques, again essentially as described in the above cited references. In a further aspect, the present disclosure provides a cell line such as a hybridoma that produces antibodies, preferably monoclonal antibodies, against DC-SIGN.

In one embodiment, the antibody of the present disclosure comprises a light chain. As used herein, "light chain" means the smaller polypeptide of an antibody molecule composed of one variable domain (VL) and one constant domain (CL), or fragments thereof. In another embodiment, the portion of the antibody comprises a heavy chain. As used herein, "heavy chain" means the larger polypeptide of an antibody molecule composed of one variable domain (VH) and three or four constant domains (CH1, CH2, CH3, and CH4), or fragments thereof.

In another embodiment, the antibody comprises a Fab portion of the antibody. As used herein, "Fab" means a monovalent antigen binding fragment of an immunoglobulin that consists of one light chain and part of a heavy chain. It can be obtained by brief papain digestion or by recombinant methods. In another embodiment, the portion of the antibody comprises a F(ab')₂ portion of the antibody. As used herein, "F(ab')₂ fragment" means a bivalent antigen binding fragment of an immunoglobulin that consists of both light chains and part of both heavy chains. It can be obtained by brief pepsin digestion or recombinant methods. In other embodiments, the antibody may be a Fab' fragment Fab expression libraries may for instance be obtained by the method of Huse et al., Science 245: 1275 (1989).

Furthermore, "humanized" antibodies may be used, for instance as described WO 98/49306. As used herein, "humanized" antibodies are those antibodies wherein amino acids outside the CDR are replaced with corresponding amino acids derived from human immunoglobulin molecules. "CDR" or "complementarity determining region" means a highly variable sequence of amino acids in the variable domain of an antibody. U.S. Patent No. 5,225,539 describes one approach for the production of humanized antibodies. Recombinant DNA technology can be used to produce a humanized antibody wherein the CDRs of a variable region of one immunoglobulin are replaced with the CDRs from an immunoglobulin with a different specificity such that the humanized antibody recognizes the desired target but is not recognized in a significant way by the human subject's immune system. Specifically, site directed mutagenesis is used to graft the CDRs onto the framework.

Other approaches for humanizing antibodies are described in U.S. Patent Nos. 5,585,089 and 5,693,761 and WO 90/07861. These antibodies have one or more CDRs and additional amino acids from a donor immunoglobulin and a framework region from an accepting human immunoglobulin. Specifically, these patents describe the preparation of a humanized antibody that binds to a receptor by combining the CDRs of a mouse monoclonal antibody with human immunoglobulin framework and constant regions. Human framework regions can be chosen to maximize homology with the mouse sequence. A computer model can be used to identify amino acids in the framework region which are likely to interact with the CDRs or the specific antigen and then mouse amino acids can be used at these positions to create the humanized antibody.

In one embodiment, the antibody includes one or more CDR domains of the antibody. In another embodiment, the antibodies utilized in the present disclosure are humanized antibodies having a light chain variable region comprising at least one CDR selected from the group consisting of amino acid sequences of SEQ ID NO: 45, 46, 56, 57, 58, 59, 60, 61 and 62. In yet another embodiment, the antibodies utilized in the present disclosure are humanized antibodies having a heavy chain variable region comprising at least one CDR selected from the group consisting of amino acid sequences of SEQ ID NO: 47, 48, 49, 50, 51, 52, 53, 54, and 55.

It is envisaged that the novel antibodies of the present disclosure will have broad applicability (i.e., besides the pharmaceutical/therapeutic uses disclosed herein). Some of these applications, which form yet another aspect of the present disclosure, will be clear to the skilled person from the disclosure herein.

Once obtained, the antibodies described above can be administered to an animal. In one embodiment, the anti-DC-SIGN antibodies reduce the immune response in an animal, in particular a human or another mammal. By binding to DC-SIGN, the antibodies impede the interaction(s) between DC-SIGN-expressing cells and ICAM-expressing cells, e.g., the interaction between a dendritic cell and a T cell. More specifically, the antibodies to DC-SIGN reduce the adhesion between a dendritic cell and a T cell by interfering with the adhesion between DC-SIGN and an ICAM receptor on the surface of a T cell.

As used herein, "ICAM receptor(s)" means both the ICAM-2 and ICAM-3 receptor, especially the ICAM-3 receptor.

By interfering with the adhesion of T cells to dendritic cells, the use of antibodies to DC-SIGN will affect dendritic cell-T cell clustering, T cell activation and other interactions that rely on contact between dendritic cells and T cells. These other interactions include both direct cell-to-cell contact or close proximity of dendritic cells and T cells.

Such further interactions include, but are not limited to, processes involved in generating an immune response, in particular during the initial stages of such a response, such as primary sensitization/activation of T lymphocytes, i.e., presentation of antigen and/or MHC-bound peptides to T cells and co-stimulation of T cells. In addition, such interactions include processes such as chemical signaling, endocytosis and transepithelial transport. For a discussion of dendritic cell-T cell interactions in general, all of which may be influenced by the compositions of the present disclosure, reference is made to the discussion below as well as to WO 95/32734 and WO 96/23882.

It is also contemplated that the present antibodies can be labeled with a toxin to DC-SIGN expressing cells. Administration of the anti-DC-SIGN antibodies labeled with toxin can then be utilized to reduce the levels of DC-SIGN expressing cells which, in some instances, can be beneficial, such as in the treatment of autoimmune disease, cancer or inflammatory diseases. In this manner, the present antibodies can also be utilized to kill or ablate DC-SIGN expressing cells in vivo. This involves administering the antibodies bonded to a cytotoxic drug (e.g., a toxin or radiation-emitting compound) to a subject requiring such treatment. Since the antibodies recognize DC-SIGN expressing cells (e.g., cancer cells or dendritic cells), any such cells to which the antibodies bind are destroyed. It is also contemplated that the present antibodies could be used to kill L-SIGN expressing cells, since the present anti-DC-SIGN antibodies have also been shown to bind to some extent to L-SIGN as well. In one embodiment, a method of treating cancer in accordance with this disclosure involves administering an effective cancer-cell killing amount of an anti-DC-SIGN antibody having a toxin bound thereto to a cancer patient. In another embodiment, a method of treating an inflammatory disease in accordance with this disclosure involves administering an effective dendritic-cell killing amount of an anti-DC-SIGN antibody having a toxin bound thereto to a patient suffering from an inflammatory disease.

Furthermore, the antibodies of the present disclosure can be used to prevent or reduce the transfer of matter from dendritic cells to T cells, such as chemicals, signaling factors such as chemokines and/or interleukins, etc., and in particular of viral particles such as HIV-1, HIV-2, SIV-1, hepatitis C virus (HCV), Ebola, SARS, cytomegalovirus (CMV), Sindbis, and Dengue. In this way, by using the antibodies of the present disclosure, not only can the initial adhesion of viral particles to dendritic cells be inhibited, but also the spread of viral infection from dendritic cells to T cells.

The antibodies of the present disclosure can not only be used to prevent viral infection of dendritic cells, but also to reduce the spread of viral infection to T cells after the dendritic cells have been infected, thereby slowing down the disease process. The antibodies may also be used to prevent, inhibit or at least delay T cell activation and thereby slow the onset and/or the progress of a viral disease such as HIV.

The antibodies of the present disclosure can therefore be used to influence the immunomodulatory ability of dendritic cells; to modulate, and in particular reduce, dendritic cell-mediated (primary) T cell responses, and/or generally to influence, and in particular inhibit, the immune system. The antibodies can be used for preventing and/or treating disorders of the immune system, as well as to prevent transplant rejection.

Some additional applications include preventing or inhibiting immune responses to specific antigens; inducing tolerance; immunotherapy; immunosuppression, i.e., to prevent transplant rejection; the treatment of auto-immune diseases such as thyroiditis, rheumatoid arthritis, systemic lupus erythematosus (SLE), multiple sclerosis and auto-immune diabetes; and the prevention or treatment of allergies.

The antibodies of the present disclosure constitute a very useful diagnostic and research tool, for use both in vitro as well as in vivo. Possible non-limiting fields of application include the study of dendritic cells and their function and interactions; the study of the immune system; the detection of dendritic cells and/or C-type lectins in cells, tissues or biological fluids such as synovial tissue and skin tissue/skin cells; as well as the study of the role dendritic cells play in biological processes or disease mechanisms, such as cancer and auto-immune diseases (including, e.g., rheumatoid arthritis).

The antibodies of the present disclosure can be used to detect the presence of (and thereby determine the expression of) DC-SIGN in or on tissues or whole cells, as well as detect the presence of DC-SIGN in other biological samples such as cell fragments or in cell preparations. The information thus obtained can then be used to determine whether the method or compositions of the present disclosure can be applied to such tissues or cells. The antibodies of the present disclosure could also be used to detect (qualitatively and/or quantitatively), isolate, purify and/or produce dendritic cells, for instance in/from biological samples, including biological fluids such as blood, plasma or lymph fluid; tissue samples or cell samples such as bone marrow, skin tissue, tumor tissues, etc; or cell cultures or cultivating media. Detection can be by suitable assays.

Assays could be used in a manner known per se for the analysis of antibodies, such as competitive inhibition assays or ELISA-type immunoassays. For instance, the antibodies could be used in combination with microscopy techniques, cell sorting techniques including flow-cytometry and fluorescence activated cell sorting (FACS), techniques based upon solid supports and/or detectable labels or markers (which can be attached to the antibodies), techniques based upon (para)magnetic beads or any other detection or assay technique known to one skilled in the art in which antibodies can be used. Such assays and kits for therein, which besides the antibodies of the present disclosure can contain additional components known for antibody-based assays, as well as manuals etc., form a further aspect of the present disclosure.

By using the antibodies of the present disclosure, dendritic cells can also be isolated and produced with higher yield and with higher specificity. In such a method, the antibodies can be used in a manner known per se for the harvesting, isolation and/or purification of cells from biological fluids using antibodies.

For a further description of the methods and known techniques in which the antibodies of the present disclosure can be used, reference is made to the general textbooks, such as Sites, et al., "Basic and clinical immunology", 8th Ed., Prentice-Hall (1994); Roitt, et al., "Immunology", 2nd. Ed., Churchill Livingstone (1994); the contents of which are incorporated by reference herein. Particular reference is made to the general uses of antibodies and techniques involved therein as mentioned in sections 2.7 to 2.17 of the general reference work by Janeway-Travers: "Immunobiology, the immune system in health and disease", Third Edition.

The present disclosure further relates to a method for the prevention or treatment of HN infections, comprising administering to a HIV infected patient or a person at risk of becoming HIV infected, a compound that can binds or bind to DC-SIGN on the surface of a dendritic cell, in such an amount that the adhesion of HIV to the dendritic cells is inhibited. It is also contemplated that the present antibodies could be used to prevent viral infection of cells having L-SIGN on their surface, since the present anti-DC-SIGN antibodies have also been shown to bind to some extent to L-SIGN as well.

Also, the present disclosure further relates to a method for the treatment of viral infections, comprising administering to an infected patient a compound that binds or can bind to DC-SIGN in such an amount that the transfer of the virus from infected dendritic cells to non-infected T cells is inhibited.

In another aspect, antibodies of the present disclosure are used to modulate, and in particular generate, increase and/or promote, an immune response in an animal, such as a human or another mammal, against a specific peptide, i.e., an antigen or combination of antigens, by presenting said antigen(s) or one or more antigenic parts thereof to dendritic cells in a form that can bind to DC-SIGN. The antigen(s) presented in this manner are internalized, i.e., they enter the dendritic cell, which then presents the antigen on its surface to the T cells, thereby causing an immune response against the antigen(s).

The phrase "a form that can bind to DC-SIGN" with respect to presentation to dendritic cells is generally meant that the antigen or antigenic fragment is attached to the anti-DC-SIGN antibodies described above. Said attachment can be by covalent binding, ligand-ligand interaction, complexing, ligation, fusion of proteins (e.g. through expression of said fusions), or by any other type of physical or chemical interaction or bond that enables the antigen to be presented to a dendritic cell in conjunction with the anti-DC-SIGN antibodies.

The antigen can be any antigen against which an immune response is to be obtained, or any part or fragment thereof. Preferably, any such part or fragment is such that it is per se capable of eliciting an immune response, such as an epitope. However, this is not required: because the fragments are directed to the dendritic cells with increased specificity or affinity by virtue of their attachment to the anti-DC-SIGN antibodies of the present disclosure, fragments that would normally be incapable of eliciting an immune response may provide an immune response when used in conjunction with anti-DC-SIGN antibodies described herein. Also, in general, using an antigen in combination with the anti-DC-SIGN antibodies may increase the potency of the antigen, i.e., provide a higher or stronger immune response per unit of antigen administered. In this way, antigens could be administered at a lower dosage and still provide sufficient immune response.

Examples of suitable antigens are cancer antigens including gp 100, g250, p53, MAGE, BAGE, GAGE, MART 1, Tyrosinase related protein 11 and Tyrosinase related protein; all of which can be used to generate an immune response against the tumor cells that contain or express said antigen. Other types of antigens that can be used in the present disclosure include essentially all antigens used in vaccines against infectious diseases, such as influenza, mumps, measles, rubella, diphtheria, tetanus, diseases due to infection with micro-organisms such as Haemophilus influenzae (e.g. type b), Neisseria, Bordetella pertussis, Polyomyletus, Influenza virus and Pneumococcus, and generally any other infection or disease against which a vaccine can be developed or can be envisaged, including also parasite, protozoan and/or viral infections such as HIV and herpes. To provide serums or vaccines, the compounds of the present disclosure may further be combined with other antigens known per se.

This aspect of the present disclosure therefore relates to compositions including a combination of: 1) an anti-DC-SIGN antibody and 2) an antigen or a fragment or part thereof attached thereto. The combination of the two may be utilized in a composition for modulating, in particular generating, increasing and/or promoting, an immune response in an animal, particularly a human or another mammal, against said antigen. This technique could be especially advantageous in cancer vaccines.

The above combinations can be in the form of a complex, a chemical substance or entity, or a fused protein or protein structure, and can be formulated and administered as a composition in a manner known to one skilled in the art. Thus, once obtained, the above antibodies to DC-SIGN in combination with a peptide may be administered to a host animal to boost T cell response and thus the immune response of the host animal.

In a further aspect the present disclosure relates to a method for modulating the immune response in an animal, in particular a human or another mammal, comprising administering to said animal antibodies to DC-SIGN in combination with a peptide, referred to herein as an "antibody/peptide construct", preferably in the form of a composition as described herein, in an amount sufficient to alter or modify an immune response. Preferably this method generates an immune response to the peptide.

Compositions for administration in accordance with the present disclosure, regardless of their intended effect, may contain one or more of the abovementioned anti-DC-SIGN antibodies, or such antibodies in combination with other compounds. In some embodiments, an antibody can be formulated with mannose, fucose or other carbohydrates, lectins and/or antibiotics such as pridamicin A, whereby a synergistic effect may be obtained.

For example, in some embodiments the anti-DC-SIGN antibodies or antibody/peptide constructs of the present disclosure may be utilized to block the binding, infection, and transmission of infectious agents including, but not limited to, viruses such as HIV, HCV, Ebola, SARS, CMV, Sindbis and Dengue; bacteria such as Helicobacter pylori, Klebsiella pneumonae, and bacteria of the Mycobacterium genus, including M. tuberculosis and M. bovis; yeast such as Candida albicans; and parasites such as Leishmania pifanoi and Schistosoma mansoni. In some embodiments, the anti-DC-SIGN antibodies also bind L-SIGN, which may be useful in blocking the binding, infection, and transmission of the infectious agents described above.

Thus, the anti-DC-SIGN antibodies or antibody/peptide constructs of the present disclosure may be utilized to treat a subject animal such as a human having an existing viral or bacterial infection. In the alternative, the anti-DC-SIGN antibodies or antibody/peptide constructs of the present disclosure may be included in vaccines to prevent infection of a subject animal such as a human by a virus.

Anti-DC-SIGN antibodies or antibody/peptide constructs of the present disclosure may also be utilized as routine diagnostics for tumor types associated with DC-SIGN expression. For example, upregulation of DC-SIGN in cancer samples could be utilized as the basis for a diagnostic tool to evaluate whether a cancer has become exposed to the immune system, since upregulation of immune receptors is expected to happen only under pressure by the immune system. Using methods known to those skilled in the art, including immunohistochemistry and/or FACS analysis, tumor biopsies may be exposed to anti-DC-SIGN antibodies or antibody/peptide constructs of the present disclosure and then analyzed for the presence of bound anti-DC-SIGN antibodies, which would be indicative of a cancer associated with DC-SIGN expression. In some embodiments the anti-DC-SIGN antibodies or antibody/peptide constructs may be provided as part of diagnostic kits for determining the presence of a cancer expressing DC-SIGN.

Anti-DC-SIGN antibodies or antibody/peptide constructs of the present disclosure may also be utilized as therapeutics for the treatment of cancers characterized by an increase in DC-SIGN expression. In one embodiment, the anti-DC-SIGN antibodies or antibody/peptide constructs of the present disclosure induce ADCC (antibody-dependent cellular cytotoxicity) or CDC (complement-dependent cytotoxicity) of tumor cells, thereby killing said cells.

The present antibodies can also be utilized to kill or ablate cancerous cells in vivo. This involves administering the antibodies bonded to a cytotoxic drug to a subject requiring such treatment. Since the antibodies recognize cancer cells, any such cells to which the antibodies bind are destroyed.

The antibodies of the present disclosure may be used to deliver a variety of cytotoxic compounds. Any cytotoxic compound can be fused to the present antibodies. The fusion can be achieved chemically or genetically (e.g., via expression as a single, fused molecule). The cytotoxic compound can be a biological, such as a polypeptide, or a small molecule. As those skilled in the art will appreciate, for small molecules, chemical fusion is used, while for biological compounds, either chemical or genetic fusion can be employed.

The antibodies of the present disclosure may be used to deliver a variety of cytotoxic drugs including therapeutic drugs; a compound emitting radiation; molecules of plant, fungal, or bacterial origin; biological proteins; and mixtures thereof. The cytotoxic drugs can be intracellularly acting cytotoxic drugs, such as short-range radiation emitters, including, for example, short-range, high-energy α-emitters. Enzymatically active toxins and fragments thereof are exemplified by diphtheria toxin A fragment, nonbinding active fragments of diphtheria toxin, exotoxin A (from Pseudomonas aeruginosa), ricin A chain, abrin A chain, modeccin A chain, α-sacrin, certain Aleurites fordii proteins, certain Dianthin proteins, Phytolacca americana proteins (PAP, PAPII and PAP-S), Morodica charantia inhibitor, curcin, crotin, Saponaria officinalis inhibitor, gelonin, mitogillin, restrictocin, phenomycin, and enomycin, for example. Procedures for preparing enzymatically active polypeptides of the immunotoxins are described in WO84/03508 and WO85/03508, which are hereby incorporated by reference. Certain cytotoxic moieties are derived from adriamycin, chlorambucil, daunomycin, methotrexate, neocarzinostatin, and platinum, for example.

Procedures for conjugating the antibodies with the cytotoxic agents have been previously described.

Alternatively, the antibody can be coupled to high energy radiation emitters, for example, a radioisotope, such as ¹³¹I, a γ-emitter, which, when localized at the tumor site, results in a killing of several cell diameters. See, e.g., S. E. Order, "Analysis, Results, and Future Prospective of the Therapeutic Use of Radiolabeled Antibody in Cancer Therapy", Monoclonal Antibodies for Cancer Detection and Therapy, R. W. Baldwin et al. (eds.), pp. 303-316 (Academic Press 1985), which is hereby incorporated by reference. Other suitable radioisotopes include α-emitters, such as ²¹²Bi, ²¹³Bi, and ²¹¹At, and β-emitters, such as ¹⁸⁶Re and ⁹⁰Y. Radiotherapy is expected to be particularly effective in connection with prostate cancer, because prostate cancer is a relatively radiosensitive tumor.

In another embodiment, the anti-DC-SIGN antibodies or antibody/peptide constructs of the present disclosure may be utilized as therapeutics to treat tumors by binding to DC-SIGN and preventing negative regulation of the immune system through DC-SIGN expressing cancer cells. By preventing this negative regulation, the immune system may proceed to eradicate the cancer cells.

The efficacy of the above treatments may be confirmed utilizing methods known to those skilled in the art, including xenograft models.

Antibodies or antibody/peptide constructs in accordance with the present disclosure which are utilized as cancer therapeutics may also be combined with any other immunomodulatory therapy, such as cancer vaccines, anti-CTLA-4, anti-CD25 or cyclophosphamide to achieve increased therapeutic efficacy in the treatment of cancer.

The compositions of the present disclosure may also contain or be used in combination with known co-inhibitory compounds, such as anti-LF3A; as well as other active principles known per se, depending upon the condition to be treated. For instance, the compositions of the present disclosure may be formulated or used in combination with immunosuppressants (i.e. for preventing transplant rejection), immunomodulants, antibiotics, auto-antigens or allergens (for instance as described in WO 95/3234 or WO 96/23882), Tumor Necrosis Factor (TNF), and anti-viral agents such as anti-HIV agents and CD4 inhibitors including CD4 directed antibodies such as Leu-3A, whereby a synergistic effect can also be obtained.

The compositions of the present disclosure can be formulated using known carriers and/or adjuvants to provide a pharmaceutical form known per se, such as a tablet, capsule, powder, freeze-dried preparation, solution for injection, etc., preferably in a unit dosage form. Such pharmaceutical formulations of antibodies, their use and administration (single or multi-dosage form), as well as carriers, excipients, adjuvants and/or formulants for use therein, are generally known in the art and are for instance described in WO 93/01820, WO 95/32734, WO 96/23882, WO 98/02456, W098/41633 and/or WO 98/49306, the contents of each of which are incorporated by reference herein. Furthermore, the formulation can be in the form of a liposome, as described in WO 93/01820.

The compositions of the present disclosure may further be packaged, for instance in vials, bottles, sachets, blisters, etc.; optionally with relevant patient information leaflets and/or instructions for use.

In all the above methods, the compounds/compositions used will be administered in a therapeutically effective amount, for which term reference is generally made to WO 93/01820, WO 95/32734 and/or WO 96/23882, the contents of which are incorporated by reference herein. The administration can be a single dose, but is preferably part of a multidose administration regimen carried out over one or more days, weeks or months.

Kits according to the present disclosure include frozen or lyophilized antibodies to be reconstituted, respectively, by thawing (optionally followed by further dilution) or by suspension in a (preferably buffered) liquid vehicle. The kits may also include buffer and/or excipient solutions (in liquid or frozen form), or buffer and/or excipient powder preparations to be reconstituted with water, for the purpose of mixing with the antibodies to produce a formulation suitable for administration as a therapeutic. Thus, preferably the kits containing the antibodies are frozen, lyophilized, pre-diluted, or pre-mixed at such a concentration that the addition of a predetermined amount of heat, water, or solution provided in the kit will result in a formulation of sufficient concentration and pH as to be effective for in vivo or in vitro use. Preferably, such a kit will also comprise instructions for reconstituting and using the antibody composition as a therapeutic. The kit may also comprise two or more component parts for the reconstituted active composition. For example, a first component can include the antibodies and the second component can include a bifunctional chelate or a therapeutic agent such as a radionuclide, which when mixed with the antibodies forms a conjugated system therewith. The above-noted buffers, excipients, and other component parts can be sold separately or together with the kit.

Furthermore, although the present disclosure is described herein with respect to antibodies to DC-SIGN, it is not excluded that other, generally similar C-type lectins, including natural variants of DC-SIGN, may also be present on dendritic cells and/or may be involved in dendritic cell - T cell interaction. Such variants will usually have a high degree of amino acid homology (more than 80% to more than 90%) with, and/or be functionally equivalent to DC-SIGN. Thus, in some cases it is anticipated that the anti-DC-SIGN antibodies of the present disclosure may also bind to such variants, thereby altering DC-T cell interaction as described above with respect to DC-SIGN.

The following non-limiting examples are provided to illustrate the present disclosure.

### EXAMPLE 1

### Library construction

Mice were immunized with immature dendritic cells obtained by maturing primary blood lymphocytes with 500 U/ml IL-4 and 800 U/ml GM-CSF for 6 days. After 3 immunizations, the spleen was harvested and homogenized in TRI reagent (Molecular Research Center). Total RNA was isolated according to the manufacturer's instruction. Messenger RNA was purified using Oligotex (Qiagen Inc., Valencia, CA). First strand cDNA was synthesized using SUPERSCRIPT First-Strand Synthesis System for RT-PCR (Invitrogen Life Technologies) according to the manufacturer's protocol. First strand cDNA was mixed with oligonucleotides: mCG1Xcm I for IgG1, mCG2asBsaJ I for IgG2a, and mCKHpa I for kappa light chain in separate tubes and digested with Xcm I (for IgG1), BsaJ I (for IgG2a) and Hpa I (for kappa light chain). The sequences of these oligonucleotides are set forth below:

| | | |
|---|---|---|
| mCG1Xcm I | 5'CTAACTCCATGGTGACCCTGGGATG3' | SEQ ID NO: 1 |
| mCG2aBsaJ I | 5'CAACTGGCTCCTCGGTGACTCTAG3' | SEQ ID NO: 2 |
| mCKHpa I | 5'CAGTGAGCAGTTAACATCTGGAGG3' | SEQ ID NO: 3 |

Quality and full digestion of the first strand cDNA was checked with PCR using primers that annealed both externally and internally to the digestion site.

Second strand cDNA synthesis was performed using primers that possessed a portion that hybridized to the framework 1 region of heavy chain and light chain genes, a restriction enzyme site, and a non-hybridizing predetermined sequence. Second strand synthesis was repeated for 20 cycles consisting of 94°C for 5 seconds, 56°C for 10 seconds, and 68°C for 2 minutes.

At the end of the cycle, oligonucleotides (TMX24mCGlnoer, TMX24mCG2anoer, and TMX24mCKnoer) for extension reactions were added on ice and the synthesized cDNAs were further extended along these oligonucleotides by incubating them at 94°C for 1 minute and then at 68°C for 2 minutes. The oligonucleotides used for the extension reaction had a portion that hybridized to the constant region of the antibody gene, a restriction enzyme site, and a non-hybridizing predetermined sequence. A nucleotide at the very 3' end of the oligonucleotide was non-hybridizing and three 3' end nucleotides were modified with phosphorthioate and 2' OMe linked propyl group on the 3' end which prevented extension along the synthesized second strand cDNA and made it protective against exo- and endonuclease activity. The sequences of these oligonucleotides are set forth below:

### TMX24mCG1noer

5'GACGTGGCCGTTGGAAGAGGAGTGCCTAGGGTTACCATGGAGTTAGTTTGGGC
AGCAGA2'OMe[U(ps)C(ps)A(ps)](propyl) 3'                    SEQ ID NO: 4

### TMX24mCG2anoer

5'GACGTGGCCGTTGGAAGAGGAGTGCCTAGGGTCATCGAGGAGCCAGTTGTATC
TCCACA2'OMe[C(ps)A(ps)U(ps)](propyl) 3'                    SEQ ID NO: 5

### TMX24mCKnoer

5'GACGACCGGCTACCAAGAGGAGTGTCCGGATGTTAACTGCTCACTGGATGGTG
GGAAGATGG2'OMe[A(ps)U(ps)U(ps)](propyl) 3'     SEQ ID NO: 6

After the extension reaction was completed, the reaction was then cooled to 4°C and cleaned by a PCR purification kit (Qiagen Inc., Valencia, CA).

Single primer amplification was then performed using primers (TMX24mH and TMX24mK) that had the same predetermined sequences used for the primers for the second strand cDNA synthesis and oligonucleotides for the extension reaction. The sequences of the primers for single amplification were selected so that they have no significant homology to the known mouse gene and were as follows:

| | | |
|---|---|---|
| TMX24mH | 5'GACGTGGCCGTTGGAAGAGGAGTG3' | SEQ ID NO: 7 |
| TMX24mK | 5'GACGACCGGCTACCAAGAGGAGTG3' | SEQ ID NO: 8 |

Amplified products were purified with PCR purification kit and digested with Xho I/Bln I (IgG1 and IgG2a) and Xba I/BspE I (kappa light chain) and cloned into a PAX313m/hG vector.

### EXAMPLE 2

### Panning

The IgG 1 and IgG2a libraries were panned on recombinant human DC-SIGN-Fc. Phage (10¹²) were incubated in 2 wells of a 96 well plate coated with 1 µg/ml DC-SIGN captured by anti-human Fc antibody. After 2 hours of incubation at 37°C, the wells were washed 3 times with phosphate buffered saline (PBS) for the 1^{st} round of panning, 5 times for the 2^{nd} round of panning and 10 times for the 3^{rd} round of planning, with 5 minute intervals between washes. Bound phage was eluted with 0.1 M HCL containing 1 mg/ml bovine serum albumin (BSA) at pH 2.2. Erythrocyte rosette (ER) cells were infected with the eluate and cultured in the presence of carbicillin and tetracycline for 2 hours before the addition of isopropylthio-β-D-galactoside (IPTG) and helper phage. Two hours later, kanamycin was added and the cells were grown overnight. The next day, cultures were spun down and phage was precipitated from the supernatant using polyethylene glycol/sodium chloride (PEG/NaCI).

### EXAMPLE 3

### Solid phase ELISA

ELISA plates were coated overnight at room temperature with 2 µg/ml of anti-human Fc in PBS. Following 3 rounds of washing with PBS containing 0.05% Tween, the plates were blocked with PBS containing 1%BSA. After 1 hour at 37°C, plates were washed 3 times and incubated for 2 hours with 500 ng/ml recombinant human DC-SIGN-Fc protein. Culture supernatants from clones grown overnight in SB containing 1µg/ml carbicillin were added for 2 hours. After 3 washes, bound antibody was detected with alkaline phosphatase conjugated anti-mouse Fab'2 antibody followed by the addition of SigmaS substrate. Color development was detected at OD 405 using a plate reader (Molecular Devices).

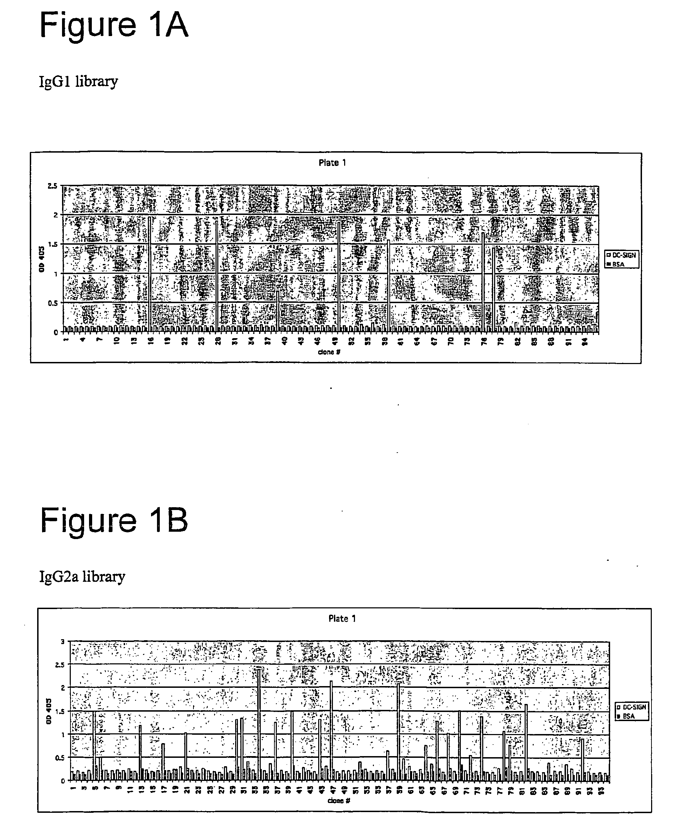

For the IgG2a library, 47/291 clones showed a 4-13-fold increase in signal to DC-SIGN over BSA. The IgG 1 library yielded 22/240 clones from screened pan 3 exhibiting a strong signal on DC-SIGN, which was up 23-fold stronger over the BSA signal. A representative example for a 96 well plate for each library is shown in Figure 1.

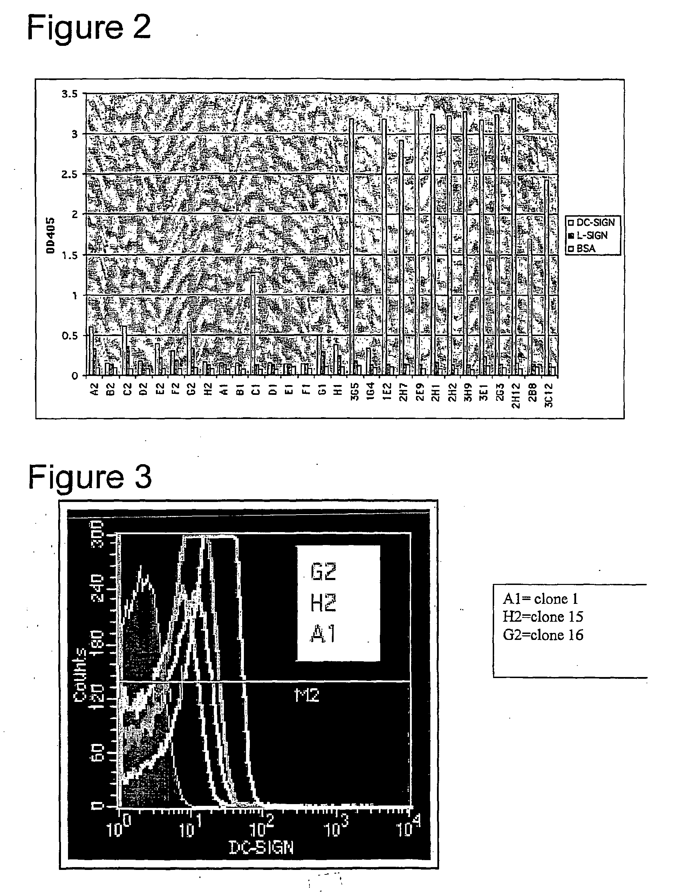

Clones showing a signal on DC-SIGN were examined for their reactivity with DC-SIGNR (L-SIGN), a highly related protein. The ELISA was performed similar to the DC-SIGN ELISA except that L-SIGN instead of DC-SIGN was coated on the plate. As shown in Figure 2, clones from the IgG1 library were highly specific for DC-SIGN. Clones from the IgG2a library generally showed a lower signal and some clones did cross-react with L-SIGN.

### EXAMPLE 4

### FACS analysis

To verify that the clones reacting with recombinant DC-SIGN also recognized DC-SIGN on the cell surface, immature dendritic cells were incubated with culture supernatant of the clones of interest and diluted 1:1 with FACS buffer (PBS containing 1% BSA and 0.1% NaN₃) for 20 minutes on ice. Cells were washed twice with FACS buffer and cell surface bound antibody was detected with a PE-conjugated anti-mouse IgG antibody. Samples were analyzed using a FACS Calibur (Becton Dickinson). The results for some representative samples are set forth in Figure 3. As seen in Figure 3, all samples recognized the protein on immature dendritic cells.

### EXAMPLE 5

### Sequences

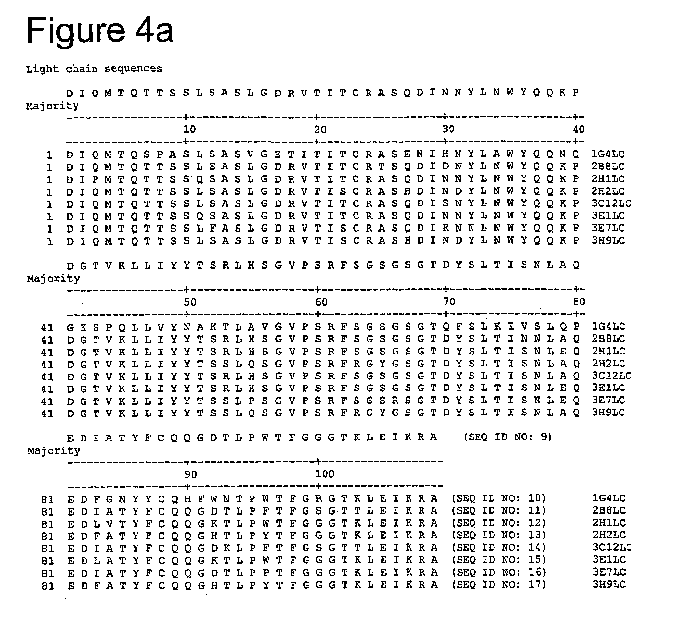

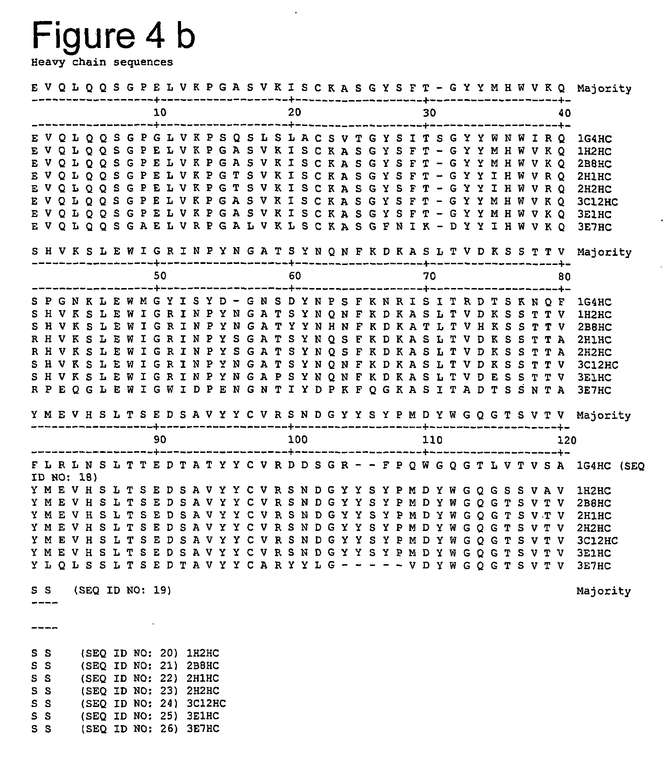

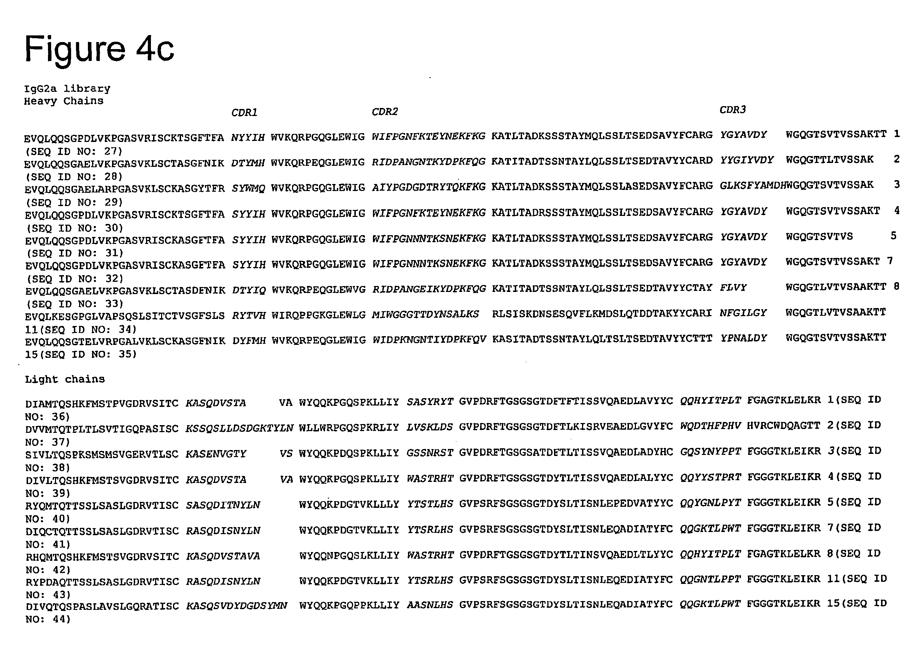

DNA from 16 clones from each library giving a positive signal in solid phase ELISA was isolated and submitted for sequencing to Retrogen, Inc. (San Diego, CA). Sequences obtained are set forth in Figures 4a-c. Only unique sequences are shown. As set forth in Figure 4a, particularly useful light chain CDR3 regions that will bind to human DC-SIGN have one of the following sequences: QHFWNTPWT (SEQ ID NO: 45); or QQGHTLPYT (SEQ ID NO: 46). As set forth in Figure 4b, particularly useful heavy chain CDR3 regions that will bind to human DC-SIGN have one of the following sequences: SNDGYYS (SEQ ID NO: 47); RYYLGVD (SEQ ID NO: 48); or DDSGRFP (SEQ ID NO: 49).
As set forth in Fig 4c, the heavy chain CDR3 regions of the antibodies that bind to human DC-SIGN may also have one of the following amino acid sequences: YGYAVDY (SEQ ID NO: 50); YYGIYVDY (SEQ ID NO: 51); FLVY (SEQ ID NO: 52); NFGILGY (SEQ ID NO: 53); YPNALDY (SEQ ID NO: 54); or GLKSFYAMDH (SEQ ID NO: 55). As also set forth in Fig 4c, the light chain CDR3 regions of the antibodies that bind to human DC-SIGN may also have one of the following amino acid sequences: QQGKTLPWT (SEQ ID NO: 56); QQGNTLPPT (SEQ ID NO: 57); QQHYITPLT (SEQ ID NO: 58); QQYGNLPYT (SEQ ID NO: 59); QQYYSTPRT (SEQ ID NO: 60); GQSYNYPPT (SEQ ID NO: 61); or WQDTHFPHV (SEQ ID NO: 62).

### EXAMPLE 6

### Competition ELISA

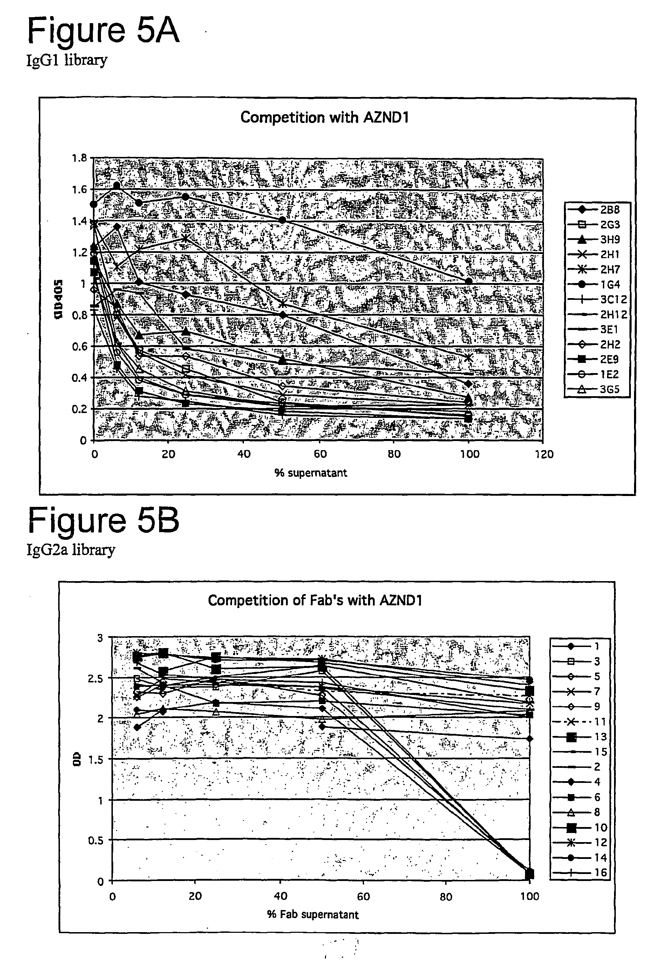

To determine whether clones reactive with DC-SIGN recognized an epitope different from AZN-D1, a known anti-DC-SIGN antibody described in WO 00/63251, a competition ELISA was performed. Plates were coated with recombinant human DC-SIGN as described above. Culture supernatants were added at various concentrations in the presence of 1 µg/ml AZN-D1. Binding of AZN-D1 was detected using an alkaline-phosphatase conjugated anti-mouse Fc antibody followed by SigmaS substrate. Color development was detected at OD 405 using a plate reader (Molecular Devices). Loss of signal indicated that the Fab and AZN-D1 competed for the same epitope. As shown in Figure 5, for the IgG1 library clones there was only weak competition with clones 1G4, 2H7 and 2B8 (2H7 and 2B8 turned out to have identical sequence). For the IgG2a library clones, clones 2,4,5,6,7,8,9,11,12,13,14 and 15 did not compete indicating that they recognized epitopes very distinct from AZN-D1. For those clones showing some competition, the epitopes recognized, while possibly still somewhat different, were close enough to AZN-D1's epitope to be blocked by the antibody.

### EXAMPLE 7

### ICAM-3/ DC-SIGN Bead assay

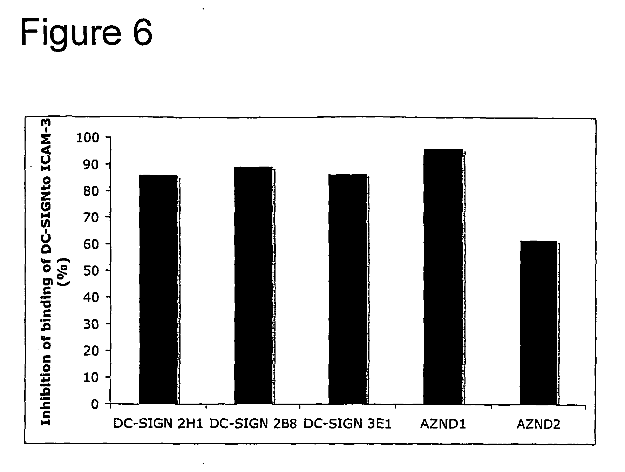

To determine whether the antibodies block interaction of ICAM-3 with DC-SIGN, a fluorescent bead assay was performed using selected clones. Carboxylate-modified TransFluorSpheres (488/645 nm, 1.0 µm; from Molecular Probes, Inc., Eugene OR) were coated with ICAM-1 Fc and ICAM-3 Fc proteins (obtained from R & D Systems, Minneapolis, MN) by incubating streptavidin-coated beads with biotinylated goat-antihuman anti-Fc F(ab)₂ for 2 hours at 37°C in PBS, 0.5% BSA. The beads were washed and incubated with ICAM-3 Fc fusion proteins (250 ng/mL) overnight at 4°C. For adhesion to ICAM coated beads, DC-SIGN-transfected K562 cells (5x10⁶/ml) were resuspended in Tris-sodium-BSA buffer (20mM Tris-HCl, pH 8.0,150mMNaCl, 1mM CaCl₂, 2mM MgCl₂, 0.5% BSA). Fifty thousand cells were preincubated with or without DC-SIGN-SIGN Fabs (20 µg/mL) for 10 minutes at room temperature in a 96-well V-shaped-bottom plate. The ICAM-coated beads (20 beads/cell) were added and the suspension was incubated for 30 minutes at 37°C.
After washing, the cells were resuspended in Tris-sodium-BSA buffer. ICAM-mediated adhesion of DC-SIGN-transfected K562 cells was measured by flow cytometry in FL-3. As shown in Figure 6, clones 2H1, 2B8 and 3E1 showed strong inhibition of ICAM binding to DC-SIGN, comparable to AZND1.

### EXAMPLE 8

### Selecting anti-DC-SIGN antibodies capable of blocking virus binding.

The selection of anti-DC-SIGN antibodies capable of blocking viral entry is accomplished by a fluorescent bead assay as described by Geijtenbeek et al.(1999), "High frequency of adhesion defects in B-lineage acute lymphoblastic leukemia" Blood 94: 754. Briefly, fifty thousand K562/DC-SIGN transfected cells are preincubated with or without anti-DC-SIGN Fabs (20 µg/mL) for 10 minutes at room temperature in a 96-well V-shaped bottom plate. Fluorescent beads (20 beads/cell) coated with viral envelope proteins, e.g., HCV E1/E2 or HIV gp120 are added and the suspension incubated for an additional 30 minutes at 37°C. After washing, the cells are resuspended in Tris-sodium-BSA buffer. The extent of blocking by anti-DC-SIGN antibodies of virus coated beads to K562/DC-SIGN cells is measured using a FACSCalibur (Becton Dickinson). The percentage of cells bound to the virus beads (negative control) in the absence of anti-DC-SIGN antibodies is set at 100 and the decrease in binding in the presence of anti-DC-SIGN antibodies expressed as % blocking.

### EXAMPLE 9

### Blocking viral entry.

After selecting anti-DC-SIGN antibodies in Example 8 above, the capacity of these anti-DC-SIGN antibodies to block viral entry is tested. DC-SIGN transfected K-562 cells are preincubated with anti-DC-SIGN antibodies for 30 minutes before adding reporter viruses expressing envelope proteins of interest or when feasible serum from virus+ or virus- donors. After 1 hour of incubation at 37°C, cells are washed 5 times with PBS and viral RNA is extracted from the cells using Qiagen's viral RNA mini spin kit (Qiagen Inc., Valencia, CA).

Viral RNA thus obtained is amplified by RT-PCR following the procedures of Gardner et al. (Gardner et al. (2003) "L-SIGI`T (CD 209L) is a liver-specific capture receptor for hepatitis C virus," Proc. Natal. Acad. Sci. USA, volume 100, page 4498), and a southern blot is performed.

### EXAMPLE 10

### Preventing viral transmission.

To test whether the anti-DC-SIGN antibodies identified in Example 8 can prevent transfer of a virus from receptor positive endothelial cells to either human T-cells or liver cells, K562/DC-SIGN cells or freshly isolated human liver sinusoidal endothelial cells (DC-SIGN+) or dendritic cells (DC-SIGN+) are incubated with anti-DC-SIGN antibodies of Example 8 for 30 minutes before adding luciferase or green fluorescent protein reporter viruses expressing envelope proteins of interest, e.g., HCV-E2, HIV gp120, Ebola (Alvarez et al. (2002) "C-type lectins DC-SIGN and L-SIGN mediate cellular entry by Ebola virus in cis and in trans," J Virol 76:6841) or Sindbis (Klimstra et al. (2003) "DC-SIGN and L-SIGN can act as attachment receptors for alphaviruses and distinguish between mosquito cell- and mammalian cell-derived viruses," J Virol 77:12022). After washing with culture medium, the cells are co-cultured with T-cells (C8166) or human liver cells (Huh-7). Reporter virus transmission is assessed either by measuring luciferase activity (relative light units) in target cell lysates or by flow cytometric analysis of GFP positive target cells in combination with suitable surface marker double staining on target cells (e.g., CD3 on T-cells).

### EXAMPLE 11

### Assessing the role of anti-DC-SIGN antibodies in blocking infection from Mycobacterium tuberculosis.

Mannosylated lipoarabinomannan (ManLAM), a carbohydrate rich structure present on the surface of *M. tuberculosis* has been reported to interact with DC-SIGN (Geijtenbeek et al. (2003) "Mycobacteria target DC-SIGN to suppress dendritic cell function," J Exp Med 197:7). High antibody titers against ManLAM are observed in people with active tuberculosis and have been shown to reduce bacterial loads in passive protection experiments (Hamasur et al. (2004) "A mycobacterial lipoarabinomannan specific monoclonal antibody and its F(ab') fragment prolong survival of mice infected with Mycobacterium tuberculosis," Clin Exp Immunol 138:30). Mycobacterial binding and infection are inhibited using anti-DC-SIGN antibodies capable of binding to ManLAM with high affinities. Strains of bacterium, e.g., M. bovis and M. tuberculosis are labeled with fluorescein isothiocyanate (FITC) as detailed in Geijtenbeek et al. (2003), supra. K562/DC-SIGN cells are incubated with FITC conjugated bacteria at a ratio of 1 to 20 in the presence or absence of anti-DC-SIGN antibodies (50 µg/ml). The extent of blocking (reduction in fluorescence) by the anti-DC-SIGN antibodies is determined by flow cytometry analysis.

### EXAMPLE 12

### Treatment of transplant patients with HCV infected liver.

If virus transmission is prevented, the antibodies to DC-SIGN can be used in a transplant setting in which donors potentially have HCV infections. To test this, mildly HCV-infected human donor liver are transplanted into immunodeficient mice such as NOD/SCID alongside with injection of primary blood lymphocytes from a healthy, HLA matched human donor. Mice are treated with antibodies over a period of one to 6 months. One to six months after transplantation, the mice are sacrificed, and the extent of HCV infection in the liver is assessed. Also, T cells are examined for infection with virus by PCR.

### EXAMPLE 13

### Identification of cancer types expressing DC-SIGN.

Malignant tissues and matching normal tissues are collected and fixed in paraformaldehyde or snap-frozen in OCT. Sections are prepared using a microtome, and the sections are stained for the presence of DC-SIGN using DC-SIGN antibodies, either directly, or by using anti-DC-SIGN antibodies conjugated to a suitable fluorochrome, such as FITC, or using a secondary fluorochrome-conjugated anti-mouse IgG. Staining results for malignant tissue that are significantly stronger than staining of normal tissue are indicative of cancer types expressing DC-SIGN.

Commercially available cell lines for those cancer types expressing DC-SIGN are then obtained and the presence of DC-SIGN is evaluated by FACS analysis. Briefly, one million cells are incubated with 1 µg of anti-DC-SIGN antibody in PBS containing 1%BSA and 0.1% NaN₃. After 30 minutes incubation, the cells are washed and incubated with fluorochrome-conjugated anti-mouse IgG for another 30 minutes before analysis with a FACSCalibur (Becton Dickinson).

### EXAMPLE 14

### Therapeutic use of anti-DC-SIGN antibodies

### Direct cell killing

Once a DC-SIGN expressing tumor type has been identified as in Example 13 above, anti-DC-SIGN antibodies are tested for their capacity to induce ADCC or CDC *in vitro.* For evaluating ADCC, target cells (tumor cells) are first labeled with ⁵¹Cr. Anti-DC-SIGN antibodies are then added at concentrations between 1-50 µg and target cell lysis by PBMC is determined after 4 hours at effector to target ratios of 1:10 - 1:100. For evaluation of CDC, tumor cells are incubated with human complement and anti-DC-SIGN antibodies. Cell killing is assessed by FACS analysis after addition of propidium iodide, a reagent that can only enter dead, but not live, cells. For antibodies that do not induce ADCC or CDC, any radiolabel or toxic reagent will remain conjugated to the antibody.

The capacity of either the naked or conjugated anti-DC-SIGN antibodies to halt tumor growth is assessed in xenograft models. Briefly, tumor cells expressing DC-SIGN are injected subcutaneously, intraperitoneally or intravenously. Animals are treated with either control or anti-DC-SIGN antibodies. Tumor growth is measured by size for subcutaneous treatment or survival time for intraperitoneal or intravenous treatment. If tumor growth in the anti-DC-SIGN antibody treated groups is reduced by more than 30% compared to the control group, the anti-DC-SIGN antibodies may be utilized as a cancer therapeutic.

### Blocking of negative regulatory interaction of cancer cells with the immune system

Antibodies blocking the interaction of DC-SIGN with immune cells are identified using the fluorescent bead assay as described in Example 8. The anti-DC-SIGN antibodies are evaluated for their therapeutic usefulness with regard to allowing the immune system to eradicate cancer cells by preventing negative regulation of the immune system through DC-SIGN expressing cancer cells.

DC-SIGN expressing tumor cells are implanted subcutaneously, intraperitoneally or intravenously into immune-deficient mice such as NOD/SCID. Mice will also receive 2 million PBMC's from healthy donors (or any number of PBMC's not sufficient to reject tumors by themselves). Tumor growth in the presence or absence of anti-DC-SIGN antibody is compared with control antibody. Tumor growth is measured by size for subcutaneous treatment or survival time for systemic tumors. If tumor growth in the anti-DC-SIGN antibody treated groups is reduced by more than 30% compared to the control group, the antibodies may be utilized as a cancer therapeutic.

It will be understood that various modifications may be made to the embodiments disclosed herein. For example, as those skilled in the art will appreciate, the specific sequences described herein can be altered slightly without necessarily adversely affecting the functionality of the antibody or antibody fragment. For instance, substitutions of single or multiple amino acids in the antibody sequence can frequently be made without destroying the functionality of the antibody or fragment. Thus, it should be understood that antibodies having a degree of homology greater than 70% to the specific antibodies described herein are within the scope of this disclosure. In particularly useful embodiments, antibodies having a homology greater than about 80% to the specific antibodies described herein are contemplated. In other useful embodiments, antibodies having a homology greater than about 90% to the specific antibodies described herein are contemplated. Therefore, the above description should not be construed as limiting, but merely as exemplifications of preferred embodiments. Those skilled in the art will envision other modifications within the scope and spirit of this disclosure.

The invention furthermore comprises the following items:
1. An antibody comprising amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, wherein the antibody binds to human DC-SIGN.
2. The antibody of item 1 further comprising a peptide attached to the antibody.
3. The antibody of item 2 wherein the peptide comprises an antigen.
4. The antibody of item 3 wherein the antigen comprises a cancer antigen.
5. A vaccine comprising the antibody of item 3.
6. A vaccine comprising the antibody of item 4.
7. A composition comprising an antibody as in item 1 and a pharmaceutically acceptable carrier.
8. An antibody as in item 1 wherein the antibody is a humanized antibody.
9. An antibody as in item 1 wherein the antibody is an scFv.
10. An antibody that binds to human DC-SIGN comprising an amino acid sequence selected from the group consisting of SNDGYYS (SEQ ID NO: 47); RYYLGVD (SEQ ID NO: 48); DDSGRFP (SEQ ID NO: 49); YGYAVDY (SEQ ID NO: 50), YYGIYVDY (SEQ ID NO: 51), FLVY (SEQ ID NO: 52), NFGILGY (SEQ ID NO: 53), YPNALDY (SEQ ID NO: 54) and GLKSFYAMDH (SEQ ID NO: 55).
11. An antibody as in item 10 wherein said amino acid sequence appears in the heavy chain CDR3 of the antibody.
12. An antibody that binds to human DC-SIGN comprising an amino acid sequence selected from the group consisting of QHFWNTPWT (SEQ ID NO: 45); QQGHTLPYT (SEQ ID NO: 46); QQGKTLPWT (SEQ ID NO: 56), QQGNTLPPT (SEQ ID NO: 57), QQHYITPLT (SEQ ID NO: 58), QQYGNLPYT (SEQ ID NO: 59), QQYYSTPRT (SEQ ID NO: 60), GQSYNYPPT (SEQ ID NO: 61) and WQDTHFPHV (SEQ ID NO: 62).
13. An antibody as in item 12 wherein said amino acid sequence appears in the light chain CDR3 of the antibody.
14. A method for interfering with the interaction of DC-SIGN expressing cells and ICAM-expressing cells comprising administering to a subject an effective immune-modulating amount of an antibody in accordance with item 1.
15. A method for generating an immune response comprising administering to a subject an effective immune-modulating amount of an antibody in accordance with item 2.
16. A method for interfering with the interaction of DC-SIGN expressing cells and ICAM-expressing cells comprising administering to a subject an effective immune-modulating amount of an antibody in accordance with item 10.
17. A method for interfering with the interaction of DC-SIGN expressing cells and ICAM-expressing cells comprising administering to a subject an effective immune-modulating amount of an antibody in accordance with item 12.
18. A method for delivering an antigen to DC-SIGN expressing cells comprising attaching said antigen to an antibody comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, wherein the antibody binds to human DC-SIGN.
19. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking binding of a virus selected from the group consisting of HIV, HCV, Ebola, SARS, CMV, Sindbis and Dengue to the cell.
20. An antibody in accordance with item 19, wherein the antibody also binds to L-SIGN.
21. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking infection of the cell by a virus selected from the group consisting of HIV, HCV, Ebola, SARS, CMV, Sindbis and Dengue.
22. An antibody in accordance with item 21, wherein the antibody also binds to L-SIGN.
23. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking transmission of a virus selected from the group consisting of HIV, HCV, Ebola, SARS, CMV, Sindbis and Dengue from the cell to another cell.
24. An antibody in accordance with item 23, wherein the antibody also binds to L-SIGN.
25. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ II7 NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking binding of a bacteria selected from the group consisting Helicobacter pylori, Klebsiella pneumonae, Mycobacteria tuberculosis and Mycobacteria Bovis to the cell.
26. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking infection of the cell by a bacteria selected from the group consisting Helicobacter pylori, Klebsiella pneumonae, Mycobacteria tuberculosis and Mycobacteria Bovis.
27. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking transmission of a bacteria selected from the group consisting Helicobacter pylori, Klebsiella pneumonae, Mycobacteria tuberculosis and Mycobacteria Bovis from the cell to another cell.
28. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking binding of a parasite selected from the group consisting of Leishmania pifanoi and Schistosoma mansoni to the cell.
29. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking infection of the cell by a parasite selected from the group consisting of Leishmania pifanoi and Schistosoma mansoni.
30. An antibody that recognizes a DC-SIGN receptor on a cell comprising an amino acid sequence having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62, the antibody being capable of effectively blocking transmission of a parasite selected from the group consisting of Leishmania pifanoi and Schistosoma mansoni from the cell to another cell.
31. A diagnostic agent for a tumor characterized by increased DC-SIGN expression comprising an antibody that recognizes a DC-SIGN receptor.
32. A diagnostic kit comprising the diagnostic agent of item 31.
33. A method for diagnosing cancer comprising:
   obtaining a tissue sample from a subject suspected of having cancer; and
   determining the degree to which the tissue sample binds with an antibody that recognizes a DC-SIGN receptor having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62,
      wherein an increase in the degree of binding compared to corresponding normal tissue indicates the presence of cancer.
34. A method as in item 33 wherein the determining step comprises staining for the presence of DC-SIGN.
35. A therapeutic agent for treating a cancer characterized by increased DC-SIGN expression comprising an antibody that recognizes a DC-SIGN receptor having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62.
36. A method for treating a cancer comprising administering to a subject a cancer cell killing amount of a composition comprising an antibody that recognizes a DC-SIGN receptor having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62.
37. The method of item 36 wherein the antibody that recognizes the DC-SIGN receptor induces antibody-dependent cellular cytotoxicity of cancer cells.
38. The method of item 36 wherein the antibody that recognizes the DC-SIGN receptor induces complement-dependent cytotoxicity of cancer cells.
39. The method of item 36 wherein the antibody that recognizes the DC-SIGN receptor prevents negative regulation of the immune system through DC-SIGN expressing cancer cells.
40. A method as in item 36 wherein the antibody is fused to a toxin.
41. A method as in item 36 wherein the antibody is fused to a high energy radiation emitter.
42. A method for treating an inflammatory disease comprising administering to a subject a dendritic cell killing amount of a composition comprising an antibody that recognizes a DC-SIGN receptor having at least 80% homology to an amino acid sequence selected from the group consisting of SEQ ID NO: 45, SEQ ID NO: 46, SEQ ID NO: 47, SEQ ID NO: 48, SEQ ID NO: 49, SEQ ID NO: 50, SEQ ID NO: 51 SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, and SEQ ID NO: 62.
43. The method of item 42 wherein the antibody that recognizes the DC-SIGN receptor induces antibody-dependent cellular cytotoxicity of dendritic cells.
44. The method of item 42 wherein the antibody that recognizes the DC-SIGN receptor induces complement-dependent cytotoxicity of dendritic cells.
45. The method of item 42 wherein the antibody that recognizes the DC-SIGN receptor prevents negative regulation of the immune system through DC-SIGN expressing dendritic cells.
46. A method as in item 42 wherein the antibody is fused to a toxin.
47. A method as in item 42 wherein the antibody is fused to a high energy radiation emitter.

## Claims

1. An antibody for use in treating cancer, wherein the antibody binds to DC-SIGN on the surface of a cancer cell and wherein the binding of the antibody to a DC-SIGN-expressing cancer cell kills the cancer cell.

2. The antibody for use of claim 1, wherein the antibody kills the cancer cell by inducing antibody-dependent cellular cytotoxicity.

3. The antibody for use of claim 1, wherein the antibody kills the cancer cell by inducing complement-dependent cytotoxicity.

4. The antibody for use of claim 1, wherein the antibody prevents negative regulation of the immune system by DC-SIGN-expressing cancer cells.

5. An antibody for use in treating an inflammatory disease or an autoimmune disease, wherein the antibody binds to DC-SIGN on the surface of a dendritic cell and wherein the binding of the antibody to a DC-SIGN-expressing dendritic cell kills the dendritic cell.

6. The antibody for use of claim 5, wherein the antibody kills the dendritic cell by inducing antibody-dependent cellular cytotoxicity.

7. The antibody for use of claim 5, wherein the antibody kills the dendritic cell by inducing complement-dependent cytotoxicity.

8. The antibody for use of claim 5, wherein the antibody prevents negative regulation of the immune system by DC-SIGN-expressing dendritic cells.

9. The antibody for use of claim 1 or 5, wherein the antibody is fused to a toxin.

10. The antibody for use of claim 1 or 5, wherein the antibody is fused to a high energy radiation emitter.

11. An antibody for use in diagnosing a cancer **characterized by** increased DC-SIGN expression, wherein the antibody binds to DC-SIGN on the surface of cancer cells.

12. The antibody for use of any one of claims 1 to 11, wherein the antibody comprises a CDR set selected from the group consisting of:
(i) a heavy chain CDR1 comprising amino acids 31 to 35 of SEQ ID NO:27; a heavy chain CDR2 comprising amino acids 50 to 66 of SEQ ID NO:27; a heavy chain CDR3 comprising amino acids 100 to 106 of SEQ ID NO:27; a light chain CDR1 comprising amino acids 24 to 34 of SEQ ID NO:36; a light chain CDR2 comprising amino acids 50 to 56 of SEQ ID NO:36; and a light chain CDR3 comprising amino acids 89 to 97 of SEQ ID NO:36;
(ii) a heavy chain CDR1 comprising amino acids 31 to 35 of SEQ ID NO:28; a heavy chain CDR2 comprising amino acids 50 to 66 of SEQ ID NO:28; a heavy chain CDR3 comprising amino acids 100 to 107 of SEQ ID NO:28; a light chain CDR1 comprising amino acids 24 to 39 of SEQ ID NO:37; a light chain CDR2 comprising amino acids 55 to 61 of SEQ ID NO:37; and a light chain CDR3 comprising amino acids 94 to 102 of SEQ ID NO:37;
(iii) a heavy chain CDR1 comprising amino acids 31 to 35 of SEQ ID NO:29; a heavy chain CDR2 comprising amino acids 50 to 66 of SEQ ID NO:29; a heavy chain CDR3 comprising amino acids 100 to 109 of SEQ ID NO:29; a light chain CDR1 comprising amino acids 24 to 34 of SEQ ID NO:38; a light chain CDR2 comprising amino acids 50 to 56 of SEQ ID NO:38; and a light chain CDR3 comprising amino acids 89 to 97 of SEQ ID NO:38;
(iv) a heavy chain CDR1 comprising amino acids 31 to 35 of SEQ ID NO:30; a heavy chain CDR2 comprising amino acids 50 to 66 of SEQ ID NO:30; a heavy chain CDR3 comprising amino acids 100 to 106 of SEQ ID NO:30; a light chain CDR1 comprising amino acids 24 to 34 of SEQ ID NO:39; a light chain CDR2 comprising amino acids 50 to 56 of SEQ ID NO:39; and a light chain CDR3 comprising amino acids 89 to 97 of SEQ ID NO:39;
(v) a heavy chain CDR1 comprising amino acids 31 to 35 of SEQ ID NO:31; a heavy chain CDR2 comprising amino acids 50 to 66 of SEQ ID NO:31; a heavy chain CDR3 comprising amino acids 100 to 106 of SEQ ID NO:31; a light chain CDR1 comprising amino acids 24 to 34 of SEQ ID NO:40; a light chain CDR2 comprising amino acids 50 to 56 of SEQ ID NO:40; and a light chain CDR3 comprising amino acids 89 to 97 of SEQ ID NO:40;
(vi) a heavy chain CDR1 comprising amino acids 31 to 35 of SEQ ID NO:32; a heavy chain CDR2 comprising amino acids 50 to 66 of SEQ ID NO:32; a heavy chain CDR3 comprising amino acids 100 to 106 of SEQ ID NO:32; a light chain CDR1 comprising amino acids 24 to 34 of SEQ ID NO:41; a light chain CDR2 comprising amino acids 50 to 56 of SEQ ID NO:41; and a light chain CDR3 comprising amino acids 89 to 97 of SEQ ID NO:41;
(vii) a heavy chain CDR1 comprising amino acids 31 to 35 of SEQ ID NO:33; a heavy chain CDR2 comprising amino acids 50 to 66 of SEQ ID NO:33; a heavy chain CDR3 comprising amino acids 100 to 103 of SEQ ID NO:33; a light chain CDR1 comprising amino acids 24 to 34 of SEQ ID NO:42; a light chain CDR2 comprising amino acids 50 to 56 of SEQ ID NO:42; and a light chain CDR3 comprising amino acids 89 to 97 of SEQ ID NO:42;
(viii) a heavy chain CDR1 comprising amino acids 31 to 35 of SEQ ID NO:34; a heavy chain CDR2 comprising amino acids 50 to 65 of SEQ ID NO:34; a heavy chain CDR3 comprising amino acids 99 to 105 of SEQ ID NO:34; a light chain CDR1 comprising amino acids 24 to 34 of SEQ ID NO:43; a light chain CDR2 comprising amino acids 50 to 56 of SEQ ID NO:43; and a light chain CDR3 comprising amino acids 89 to 97 of SEQ ID NO:43; and
(ix) a heavy chain CDR1 comprising amino acids 31 to 35 of SEQ ID NO:35; a heavy chain CDR2 comprising amino acids 50 to 66 of SEQ ID NO:35; a heavy chain CDR3 comprising amino acids 100 to 106 of SEQ ID NO:35; a light chain CDR1 comprising amino acids 24 to 38 of SEQ ID NO:44; a light chain CDR2 comprising amino acids 54 to 60 of SEQ ID NO:44; and a light chain CDR3 comprising amino acids 93 to 101 of SEQ ID NO:44.

13. The antibody for use of any one of claims 1 to 11, wherein the antibody comprises a CDR set selected from the group consisting of:
(i) a heavy chain CDR1 comprising amino acids 31 to 35 of SEQ ID NO:19; a heavy chain CDR2 comprising amino acids 50 to 66 of SEQ ID NO:19; a heavy chain CDR3 comprising amino acids 99 to 110 of SEQ ID NO:19; a light chain CDR1 comprising amino acids 24 to 34 of SEQ ID NO:9; a light chain CDR2 comprising amino acids 50 to 56 of SEQ ID NO:9; and a light chain CDR3 comprising amino acids 89 to 97 of SEQ ID NO:9;
(ii) a heavy chain CDR1 comprising amino acids 31 to 36 of SEQ ID NO:18; a heavy chain CDR2 comprising amino acids 51 to 66 of SEQ ID NO:18; a heavy chain CDR3 comprising amino acids 99 to 106 of SEQ ID NO:18; a light chain CDR1 comprising amino acids 24 to 34 of SEQ ID NO:10; a light chain CDR2 comprising amino acids 50 to 56 of SEQ ID NO:10; and a light chain CDR3 comprising amino acids 89 to 97 of SEQ ID NO:10;
(iii) a heavy chain CDR1 comprising amino acids 31 to 35 of SEQ ID NO:21; a heavy chain CDR2 comprising amino acids 50 to 66 of SEQ ID NO:21; a heavy chain CDR3 comprising amino acids 99 to 110 of SEQ ID NO:21; a light chain CDR1 comprising amino acids 24 to 34 of SEQ ID NO:11; a light chain CDR2 comprising amino acids 50 to 56 of SEQ ID NO:11; and a light chain CDR3 comprising amino acids 89 to 97 of SEQ ID NO:11;
(iv) a heavy chain CDR1 comprising amino acids 31 to 35 of SEQ ID NO:22; a heavy chain CDR2 comprising amino acids 50 to 66 of SEQ ID NO:22; a heavy chain CDR3 comprising amino acids 99 to 110 of SEQ ID NO:22; a light chain CDR1 comprising amino acids 24 to 34 of SEQ ID NO:12; a light chain CDR2 comprising amino acids 50 to 56 of SEQ ID NO:12; and a light chain CDR3 comprising amino acids 89 to 97 of SEQ ID NO:12;
(v) a heavy chain CDR1 comprising amino acids 31 to 35 of SEQ ID NO:23; a heavy chain CDR2 comprising amino acids 50 to 66 of SEQ ID NO:23; a heavy chain CDR3 comprising amino acids 99 to 110 of SEQ ID NO:23; a light chain CDR1 comprising amino acids 24 to 34 of SEQ ID NO:13; a light chain CDR2 comprising amino acids 50 to 56 of SEQ ID NO:13; and a light chain CDR3 comprising amino acids 89 to 97 of SEQ ID NO:13;
(vi) a heavy chain CDR1 comprising amino acids 31 to 35 of SEQ ID NO:24; a heavy chain CDR2 comprising amino acids 50 to 66 of SEQ ID NO:24; a heavy chain CDR3 comprising amino acids 99 to 110 of SEQ ID NO:24; a light chain CDR1 comprising amino acids 24 to 34 of SEQ ID NO:14; a light chain CDR2 comprising amino acids 50 to 56 of SEQ ID NO:14; and a light chain CDR3 comprising amino acids 89 to 97 of SEQ ID NO:14;
(vii) a heavy chain CDR1 comprising amino acids 31 to 35 of SEQ ID NO:25; a heavy chain CDR2 comprising amino acids 50 to 66 of SEQ ID NO:25; a heavy chain CDR3 comprising amino acids 99 to 110 of SEQ ID NO:25; a light chain CDR1 comprising amino acids 24 to 34 of SEQ ID NO:15; a light chain CDR2 comprising amino acids 50 to 56 of SEQ ID NO:15; and a light chain CDR3 comprising amino acids 89 to 97 of SEQ ID NO:15; and
(viii) a heavy chain CDR1 comprising amino acids 31 to 35 of SEQ ID NO:26; a heavy chain CDR2 comprising amino acids 50 to 66 of SEQ ID NO:26; a heavy chain CDR3 comprising amino acids 99 to 105 of SEQ ID NO:26; a light chain CDR1 comprising amino acids 24 to 34 of SEQ ID NO:16; a light chain CDR2 comprising amino acids 50 to 56 of SEQ ID NO:16; and a light chain CDR3 comprising amino acids 89 to 97 of SEQ ID NO:16.

14. The antibody for use of any one of claims 1 to 13, wherein the antibody is a humanized antibody.

15. A diagnostic kit comprising the antibody of claim 11.

16. A method for diagnosing cancer, the method comprising: providing a tissue sample obtained from a subject suspected of having cancer, and determining the degree to which the tissue sample binds with an antibody that recognizes DC-SIGN, wherein an increase in the degree of binding of the antibody to the tissue sample, as compared to the degree of binding of the antibody to the corresponding normal tissue, indicates the presence of cancer.

17. The method of claim 16, wherein the determining step comprises staining for the presence of DC-SIGN.

18. The method of claim 16 or 17, wherein the antibody comprises a CDR set selected from the group consisting of:
(i) a heavy chain CDR1 comprising amino acids 31 to 35 of SEQ ID NO:27; a heavy chain CDR2 comprising amino acids 50 to 66 of SEQ ID NO:27; a heavy chain CDR3 comprising amino acids 100 to 106 of SEQ ID NO:27; a light chain CDR1 comprising amino acids 24 to 34 of SEQ ID NO:36; a light chain CDR2 comprising amino acids 50 to 56 of SEQ ID NO:36; and a light chain CDR3 comprising amino acids 89 to 97 of SEQ ID NO:36;
(ii) a heavy chain CDR1 comprising amino acids 31 to 35 of SEQ ID NO:28; a heavy chain CDR2 comprising amino acids 50 to 66 of SEQ ID NO:28; a heavy chain CDR3 comprising amino acids 100 to 107 of SEQ ID NO:28; a light chain CDR1 comprising amino acids 24 to 39 of SEQ ID NO:37; a light chain CDR2 comprising amino acids 55 to 61 of SEQ ID NO:37; and a light chain CDR3 comprising amino acids 94 to 102 of SEQ ID NO:37;
(iii) a heavy chain CDR1 comprising amino acids 31 to 35 of SEQ ID NO:29; a heavy chain CDR2 comprising amino acids 50 to 66 of SEQ ID NO:29; a heavy chain CDR3 comprising amino acids 100 to 109 of SEQ ID NO:29; a light chain CDR1 comprising amino acids 24 to 34 of SEQ ID NO:38; a light chain CDR2 comprising amino acids 50 to 56 of SEQ ID NO:38; and a light chain CDR3 comprising amino acids 89 to 97 of SEQ ID NO:38;
(iv) a heavy chain CDR1 comprising amino acids 31 to 35 of SEQ ID NO:30; a heavy chain CDR2 comprising amino acids 50 to 66 of SEQ ID NO:30; a heavy chain CDR3 comprising amino acids 100 to 106 of SEQ ID NO:30; a light chain CDR1 comprising amino acids 24 to 34 of SEQ ID NO:39; a light chain CDR2 comprising amino acids 50 to 56 of SEQ ID NO:39; and a light chain CDR3 comprising amino acids 89 to 97 of SEQ ID NO:39;
(v) a heavy chain CDR1 comprising amino acids 31 to 35 of SEQ ID NO:31; a heavy chain CDR2 comprising amino acids 50 to 66 of SEQ ID NO:31; a heavy chain CDR3 comprising amino acids 100 to 106 of SEQ ID NO:31; a light chain CDR1 comprising amino acids 24 to 34 of SEQ ID NO:40; a light chain CDR2 comprising amino acids 50 to 56 of SEQ ID NO:40; and a light chain CDR3 comprising amino acids 89 to 97 of SEQ ID NO:40;
(vi) a heavy chain CDR1 comprising amino acids 31 to 35 of SEQ ID NO:32; a heavy chain CDR2 comprising amino acids 50 to 66 of SEQ ID NO:32; a heavy chain CDR3 comprising amino acids 100 to 106 of SEQ ID NO:32; a light chain CDR1 comprising amino acids 24 to 34 of SEQ ID NO:41; a light chain CDR2 comprising amino acids 50 to 56 of SEQ ID NO:41; and a light chain CDR3 comprising amino acids 89 to 97 of SEQ ID NO:41;
(vii) a heavy chain CDR1 comprising amino acids 31 to 35 of SEQ ID NO:33; a heavy chain CDR2 comprising amino acids 50 to 66 of SEQ ID NO:33; a heavy chain CDR3 comprising amino acids 100 to 103 of SEQ ID NO:33; a light chain CDR1 comprising amino acids 24 to 34 of SEQ ID NO:42; a light chain CDR2 comprising amino acids 50 to 56 of SEQ ID NO:42; and a light chain CDR3 comprising amino acids 89 to 97 of SEQ ID NO:42;
(viii) a heavy chain CDR1 comprising amino acids 31 to 35 of SEQ ID NO:34; a heavy chain CDR2 comprising amino acids 50 to 65 of SEQ ID NO:34; a heavy chain CDR3 comprising amino acids 99 to 105 of SEQ ID NO:34; a light chain CDR1 comprising amino acids 24 to 34 of SEQ ID NO:43; a light chain CDR2 comprising amino acids 50 to 56 of SEQ ID NO:43; and a light chain CDR3 comprising amino acids 89 to 97 of SEQ ID NO:43; and
(ix) a heavy chain CDR1 comprising amino acids 31 to 35 of SEQ ID NO:35; a heavy chain CDR2 comprising amino acids 50 to 66 of SEQ ID NO:35; a heavy chain CDR3 comprising amino acids 100 to 106 of SEQ ID NO:35; a light chain CDR1 comprising amino acids 24 to 38 of SEQ ID NO:44; a light chain CDR2 comprising amino acids 54 to 60 of SEQ ID NO:44; and a light chain CDR3 comprising amino acids 93 to 101 of SEQ ID NO:44.

19. The method of claim 16 or 17, wherein the antibody comprises a CDR set selected from the group consisting of:
(i) a heavy chain CDR1 comprising amino acids 31 to 35 of SEQ ID NO:19; a heavy chain CDR2 comprising amino acids 50 to 66 of SEQ ID NO:19; a heavy chain CDR3 comprising amino acids 99 to 110 of SEQ ID NO:19; a light chain CDR1 comprising amino acids 24 to 34 of SEQ ID NO:9; a light chain CDR2 comprising amino acids 50 to 56 of SEQ ID NO:9; and a light chain CDR3 comprising amino acids 89 to 97 of SEQ ID NO:9;
(ii) a heavy chain CDR1 comprising amino acids 31 to 36 of SEQ ID NO:18; a heavy chain CDR2 comprising amino acids 51 to 66 of SEQ ID NO:18; a heavy chain CDR3 comprising amino acids 99 to 106 of SEQ ID NO:18; a light chain CDR1 comprising amino acids 24 to 34 of SEQ ID NO:10; a light chain CDR2 comprising amino acids 50 to 56 of SEQ ID NO:10; and a light chain CDR3 comprising amino acids 89 to 97 of SEQ ID NO:10;
(iii) a heavy chain CDR1 comprising amino acids 31 to 35 of SEQ ID NO:21; a heavy chain CDR2 comprising amino acids 50 to 66 of SEQ ID NO:21; a heavy chain CDR3 comprising amino acids 99 to 110 of SEQ ID NO:21; a light chain CDR1 comprising amino acids 24 to 34 of SEQ ID NO:11; a light chain CDR2 comprising amino acids 50 to 56 of SEQ ID NO:11; and a light chain CDR3 comprising amino acids 89 to 97 of SEQ ID NO:11;
(iv) a heavy chain CDR1 comprising amino acids 31 to 35 of SEQ ID NO:22; a heavy chain CDR2 comprising amino acids 50 to 66 of SEQ ID NO:22; a heavy chain CDR3 comprising amino acids 99 to 110 of SEQ ID NO:22; a light chain CDR1 comprising amino acids 24 to 34 of SEQ ID NO:12; a light chain CDR2 comprising amino acids 50 to 56 of SEQ ID NO:12; and a light chain CDR3 comprising amino acids 89 to 97 of SEQ ID NO:12;
(v) a heavy chain CDR1 comprising amino acids 31 to 35 of SEQ ID NO:23; a heavy chain CDR2 comprising amino acids 50 to 66 of SEQ ID NO:23; a heavy chain CDR3 comprising amino acids 99 to 110 of SEQ ID NO:23; a light chain CDR1 comprising amino acids 24 to 34 of SEQ ID NO:13; a light chain CDR2 comprising amino acids 50 to 56 of SEQ ID NO:13; and a light chain CDR3 comprising amino acids 89 to 97 of SEQ ID NO:13;
(vi) a heavy chain CDR1 comprising amino acids 31 to 35 of SEQ ID NO:24; a heavy chain CDR2 comprising amino acids 50 to 66 of SEQ ID NO:24; a heavy chain CDR3 comprising amino acids 99 to 110 of SEQ ID NO:24; a light chain CDR1 comprising amino acids 24 to 34 of SEQ ID NO:14; a light chain CDR2 comprising amino acids 50 to 56 of SEQ ID NO:14; and a light chain CDR3 comprising amino acids 89 to 97 of SEQ ID NO:14;
(vii) a heavy chain CDR1 comprising amino acids 31 to 35 of SEQ ID NO:25; a heavy chain CDR2 comprising amino acids 50 to 66 of SEQ ID NO:25; a heavy chain CDR3 comprising amino acids 99 to 110 of SEQ ID NO:25; a light chain CDR1 comprising amino acids 24 to 34 of SEQ ID NO:15; a light chain CDR2 comprising amino acids 50 to 56 of SEQ ID NO:15; and a light chain CDR3 comprising amino acids 89 to 97 of SEQ ID NO:15; and
(viii) a heavy chain CDR1 comprising amino acids 31 to 35 of SEQ ID NO:26; a heavy chain CDR2 comprising amino acids 50 to 66 of SEQ ID NO:26; a heavy chain CDR3 comprising amino acids 99 to 105 of SEQ ID NO:26; a light chain CDR1 comprising amino acids 24 to 34 of SEQ ID NO:16; a light chain CDR2 comprising amino acids 50 to 56 of SEQ ID NO:16; and a light chain CDR3 comprising amino acids 89 to 97 of SEQ ID NO:16.

20. The method of any one of claims 16 to 19, wherein the antibody is a humanized antibody.

21. A pharmaceutical composition comprising: (i) the antibody of any one of claims 1 to 14; and (ii) a pharmaceutically-acceptable carrier.
